# EUROPEAN PATENT SPECIFICATION

(11) **EP 3 139 171 B1**
(45) Date of publication and mention of the grant of the patent: **12.02.2020**
(21) Application number: 15188615.7
(22) Date of filing: 06.10.2015
(51) Int. Cl.: G01N 33/68, G01N 33/58

(54) **NOVEL METHODS AND KITS FOR DETECTING OF UREA CYCLE DISORDERS USING MASS SPECTROMETRY**
NEUARTIGE VERFAHREN UND KITS ZUR DETEKTION VON HARNSTOFFZYKLUSDEFEKTEN MITTELS MASSENSPEKTROMETRIE
NOUVEAUX PROCÉDÉS ET KITS DE DÉTECTION DE TROUBLES DE CYCLE D'URÉE UTILISANT LA SPECTROMÉTRIE DE MASSE

(30) Priority: 02.09.2015 EP 15183561
(43) Date of publication of application: 08.03.2017
(73) Proprietor: Labsystems Diagnostics Oy, 01720 Vantaa (FI)
(72) Inventor: CARRARD, Géraldine, 00180 Helsinki (FI); FINGERHUT, Ralph, D-86911 Diessen (DE)
(74) Representative: Lecca, Patricia S.

(56) References cited:
- WO-A2-2015/109263
- Anonymous: "Cell-Free Protein Expression for Generating Stable Isotope-Labeled Proteins | Thermo Fisher Scientific", , 10 November 2012 (2012-11-10), XP055275448, Retrieved from the Internet: URL:https://www.thermofisher.com/nl/en/hom e/life-science/protein-biology/protein-bio logy-learning-center/protein-biology-resou rce-library/protein-biology-application-no tes/cell-free-protein-expression-generatin g-stable-isotope-labeled-proteins.html [retrieved on 2016-05-25]
- ANDERSON L W ET AL: "Glutamine and glutamate: Automated quantification and isotopic enrichments by gas chromatography/mass spectrometry", ANALYTICAL BIOCHEMISTRY, ELSEVIER, AMSTERDAM, NL, vol. 163, no. 2, 1 June 1987 (1987-06-01), pages 358-368, XP024818315, ISSN: 0003-2697, DOI: 10.1016/0003-2697(87)90236-3 [retrieved on 1987-06-01]
- Bhagwat Prasad ET AL: "Comparison of Heavy Labeled (SIL) Peptide versus SILAC Protein Internal Standards for LC-MS/MS Quantification of Hepatic Drug Transporters", INTERNATIONAL JOURNAL OF PROTEOMICS 2011 HINDAWI PUBLISHING CORPORATION USA, vol. 2014, 1 January 2014 (2014-01-01), pages 1-11, XP055479924, ISSN: 2090-2166, DOI: 10.1155/2014/451510
- NEMKOV TRAVIS ET AL: "Three-minute method for amino acid analysis by UHPLC and high-resolution quadrupole orbitrap mass spectrometry", AMINO ACIDS, SPRINGER VERLAG, AU, vol. 47, no. 11, 10 June 2015 (2015-06-10) , pages 2345-2357, XP035573845, ISSN: 0939-4451, DOI: 10.1007/S00726-015-2019-9 [retrieved on 2015-06-10]
- PIRAUD MONIQUE ET AL: "Ion-pairing reversed-phase liquid chromatography/electrospray ionization mass spectrometric analysis of 76 underivatized amino acids of biological interest: a new tool for the diagnosis of inherited disorders of amino acid metabolism", RAPID COMMUNICATIONS IN MASS SPECTROMETRY, vol. 19, no. 12, 2005, pages 1587-1602, ISSN: 0951-4198

## Description

### FIELD OF THE INVENTION

Methods, reagents and kits for high throughput screening and analysis of metabolic disorders using liquid chromatography mass spectrometry (LC-MS) are disclosed. The metabolic disorders can be amino acid, organic acid or fatty acid oxidation disorders, and particularly urea cycle disorders or defiencies, hyperammoneamia, argininosuccinic aciduria, and/or Hyperornithinemia-hyperammonemia-homocitrullinuria (HHH). The methods, reagents, and kits are particularly useful for conducting a plurality of in vitro screening tests in newborns and detecting a panel of metabolic disorders at high speeds, for confirmation and/or follow up of the same diseases.

### BACKGROUND OF THE INVENTION

Screening for biological disorders, in particular newborn screening (NBS) for these disorders, is currently performed using a variety of methods depending on the particular disorder screened. Amino acid and acylcarnitine analysis for NBS is currently performed by many parties using electrospray ionization coupled with tandem mass spectrometry (ESI-MS/MS). Liquid chromatography (LC) coupled with mass spectrometry (MS) has also been used for NBS.

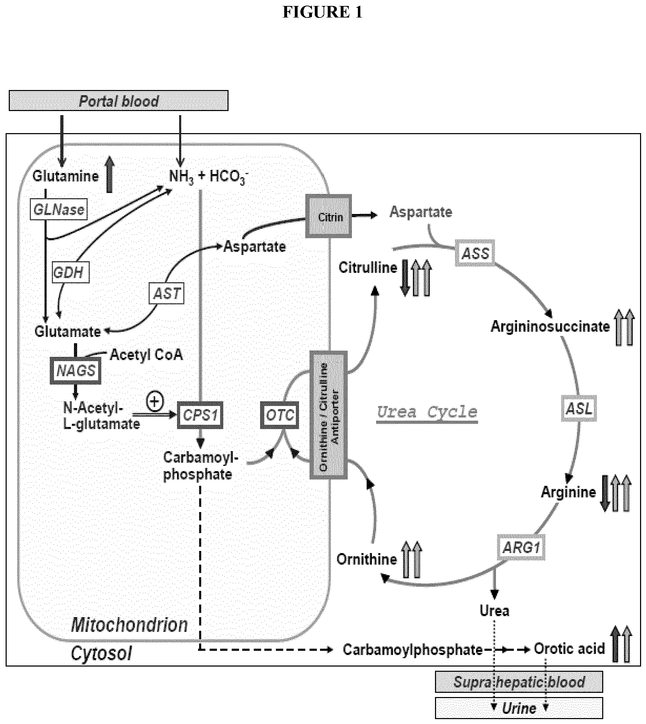

Particular newborn metabolic deficiencies to be detected in newborns are urea cycle deficiencies. The urea cycle is a metabolic pathway for disposal of the toxic metabolite ammonia and surplus nitrogen as urea in mammals. The urea cycle plays an important role in prevention of the accumulation of toxic nitrogenous compounds and further contains several of the biochemical reactions required for the *de novo* biosynthesis and degradation of arginine. As shown in Figure 1, the urea cycle is catalyzed by five enzymes: carbamoyl phosphate synthetase 1 (CPS1), ornithine transcarbamoylase (OTC), argininosuccinate synthetase (ASS), argininosuccinate lyase (ASL) and arginase (ARG1). The first two enzymes of the urea cycle are located within the mitochondrial matrix and the remaining three enzymes are cytosolic. CPS1 promotes the formation of carbamoyl phosphate from ammonium ions and carbon dioxide in the first step. The next reaction is catalyzed by OTC which transfers a carbamoyl group from carbamoyl phosphate to ornithine to form citrulline. ASS is involved in the formation of argininosuccinate from citrulline and aspartate in the third step and ASL facilitates the formation of arginine and fumarate from argininosuccinate in the fourth step. ARG1 catalyzes the formation of ornithine and urea from arginine in the fifth step. In addition, N-acetyl glutamate synthetase (NAGS) catalyses the formation of N-acetyl glutamate which activates the CPS1.

Interruptions in the metabolic pathway for urea synthesis are caused by the deficiency or inactivity of any one of several enzymes involved in specific steps in the cascade. A defect in the ureageneic pathway has two consequences: arginine becomes an essential amino acid (except in arginase deficiency, where the enzyme defect results in a failure of degradation of arginine) and nitrogen atoms accumulate in a variety of molecules the pattern of which varies according to the specific enzymatic defect although plasma levels of ammonia and glutamine are increased in all urea cycle disorders not under metabolic control.

Urea cycle disorders (UCD) include: (a) carbamyl phosphate synthetase deficiency (CPSD or CPS1), (b) N-acetyl glutamate synthetase deficiency (NAGS), (c) ornithine transcarbamylase deficiency (OTCD), (d) argininosuccinic acid synthetase deficiency (ASD), (e) argininosuccinate lyase deficiency or Citrullinemia type 1 (ALD or ASS1), and (f) arginase deficiency (ARG1). Except for OTCD, which is an X-linked generic disorder, urea cycle disorders are inherited in an autosomal recessive way. Each of these diseases represents a defect in the biosynthesis of one of the normally expressed enzymes of the urea cycle and is characterized by signs and symptoms induced by the accumulation of precursors of urea, primarilly ammonia and secondary glutamine.

The common pathologic sequelae of these clinical disorders are the extreme elevation of the plasma ammonia level (hyperammonemia). Severe urea cycle disorders are characterized by plasma ammonia level of about 2000 to 2500 micrograms/dL. Detection of hyperammonemia is most important for early diagnosis and effective treatment. Typically associated with this increase in ammonia buildup are acute episodes of vomiting, lethargy, convulsions and abnormal liver enzyme levels. Exposure to high levels of plasma ammonia is fatal typically following a period of lethargy, convulsions and coma. Even treated, protracted severe hyperammonemia leads to mental and physical retardation.

There is an existing clinical situation that challenges the introduction of universal neonatal screening for UCDs. At the mild end of the spectrum, there are patients described with a late-onset of disease with only a single, few or even absence of symptom(s) and only a biochemical phenotype. These patients were, for instance in the case of ASS deficiency, described as suffering from mild citrullinemia type 1, a condition allelic to classical citrullinemia type 1, but nevertheless much milder and with less if any need for medical intervention. Such patients were often identified in neonatal screening programs and it has been discussed whether the mild course would result from early detection and initiation of treatment or from a relevant residual enzyme or transporter function. It has been suggested that metabolites and/or mutation analysis may help to identify attenuated patients in an attempt to avoid stigmatisation of non-diseases, potentially unnecessary treatment and unnecessary anxiety to parents but no definitive solution have been offered so far.

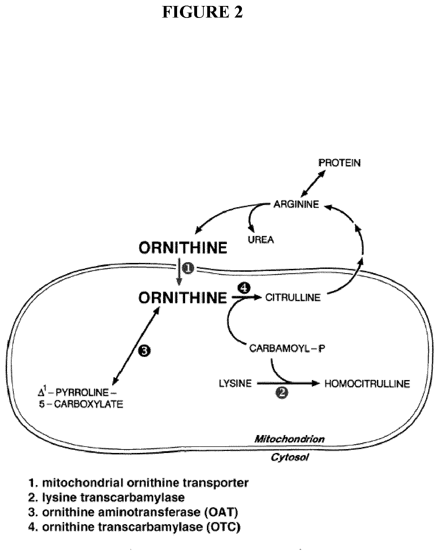

In the case of ASS deficiency (citrullinemia) and ARG1 deficiency (hyperargininemia), there is a significant accumulation of the respective substrates (citrulline and arginine) in both blood and urine. In ASL deficiency, the substrate argininosuccinate does not accumulate in blood in any appreciable amount because of the low renal threshold. Some citrulline, however, does accumulate in blood and argininosuccinate is excreted in large quantities in the urine. Attempts to develop UCDs screening tests which are based on the direct measurement of enzymes or accumulated substrates in blood or urine are limited to these last three steps in the cycle. Indeed, for the first two steps, e.g., NAGS, CPS1 and OTC, the enzymes are restricted to the mitochondria and there is no substrate accumulation (Figure 2). There is one factor common to all five enzyme deficiencies in this pathway and that is the presence of hyperammonemia. However, technically, the direct measurement of ammonia in a dried blood spot is nearly impossible.

Using arginine and citrulline as markers, ASSD and ASLD have been included in the expanded newborn screening programs in some states in the US (California, Massachusetts, Michigan, New York, Newark, Wisconsin) since 2001. Using citrulline as a marker, ASSD and ASLD have been screened for as part of the 'Recommended Uniform Screening Panel' in all of the United States since 2006. Published data from 6,077,736 births (covering years from 2001 to 2012 for different states) resulted in a cumulative incidence of 1 in 117,000 newborns for the two disorders.

One of the most common and severe defect of the urea cycle, OTC deficiency, was considered for inclusion in the panel, but it did not meet the assigned evaluation criteria, due to the lack of a screening test that had been validated in the general newborn population. OTC deficiency, an X-linked disorder, has a wide range of clinical variability and can present in a severe neonatal-onset form that is life threatening within the first few days after birth or as a late-onset, typically milder, form of the disease. Female carriers can also experience symptoms related to increased ammonia levels. The laboratory indications for OTC deficiency are elevated concentrations of glutamine and ammonia, low citrulline, and elevated excretion of orotic acid in the urine. NBS laboratories have thus attempted to use low citrulline and several ratios to identify infants at risk for OTC deficiency, but have had so far limited success.

Highlighting the difficulty to use low citrulline as a marker for the detection of proximal UCDs, a study in Tuscany applying LC-MS/MS was performed between 2001 and 2008. The authors concluded that hypocitrullinemia is not a reliable marker for OTCD newborn screening, especially for late-onset forms that may exhibit normal citrulline levels. Low citrulline concentrations may also be found in other metabolic disorders further challenging its use as screening marker. In a recent UCD guideline, it was therefore concluded that NBS for proximal disorders cannot currently be recommended, but it may be considered for the distal UCDs.

Orotic acid though often elevated in the urine of patients with OTC deficiency, cannot be used reliably as a marker in blood. Despite the fact that all UCDs affect the function of the urea cycle and therefore lead to hyperammonemia, their biochemical profile is very different.

Several other caveats regarding newborn screening for urea cycle defects are that CPS1 deficiency, OTC deficiency, and NAGS deficiency currently cannot be reliably detected. Furthermore, although hyperargininemia or ARG1 deficiency has been detected by these methods, newborn screening cannot be expected to reliably detect all cases. Even in UCDs detectable by newborn screening, neonates are often symptomatic prior to availability of the screening results; thus a high level of clinical suspicion on the part of healthcare providers is necessary. With the currently available techniques for detecting UCDs, the sensitivity and specificity of such screening is not absolute.

Since the direct measurement of ammonia is not feasible in NBS, glutamine would be another metabolite that is generally elevated in UCDs. However numbers of difficulties have been reported so far for using glutamine as a marker for UCDs. Indeed, glutamine is highly unstable in plasma and serum, and thus spontaneously convert into glutamate and pyroglutamate, which formations lead to false low glutamine levels, rendering glutamine currently not a suitable screening parameter for NBS using LC-MS.

The use of mass spectrometry (MS) in clinical laboratories has very much increased with time. This development is obviously due to great advances in mass spectrometry applications in the last fifteen years. Mass spectrometry permits a very rapid measurement of different metabolites in different biological specimens using filter paper spots or directly in different biological fluids. Because of its high sensitivity, this technique can be used for qualitative and quantitative analysis of many analytes or metabolites such as amino acids and acylcarnitines, homocysteine, orotic acid, succinylacetone etc., with appropriate internal standards. Particularly, MS is extensively used for analysis of metabolites from dried blood spots taken at birth (Guthrie-cards) but among the detected metabolites those due to UCDs are not effectively detected because the defects discussed in the earlier paragraphs.

The inclusion of UCD screening into newborn screening (NBS) is highly desirable. However, it is hampered by the fact that there is not a specific marker for every single UCD, and by the fact that so far the common feature of UCDs, e.g., hyperammonemia, is not directly detectable in dried blood spots (DBS), and by the fact that the detection of secondary elevations of glutamine seemed not feasible, because of the proposed instability of glutamine in DBS.

Hence, the aim of the present invention is to provide an analytical method that could allow the determination of metabolites correctly indicative of UCDs, particularly of glutamine, along with the determination of other metabolites that are commonly determined for metabolites screening, especially those screening performed on dried blood spots taken at birth.

The present disclosure thus provides reliable methods and kits for the simultaneous detection of lysine and glutamine from a sample in multiple reaction monitoring (MRM) with a second-tier UPLC method for the separation and a specific quantitation of glutamine. Thus the present invention makes it feasible to detect UCDs using novel markers or novel combination of markers, which were not achievable by the methods reported in prior art.

Such methods and kits may be advantageously combined with the measurement of all specific amino acids (arginine, argininosuccinic acid, citrulline, ornithine, and proline), n-acetyl-glutamate, and orotic acid. This novel combination and method and reagent kit allow for very fast and reliable determination and detection of a plurality of UCDs in newborns using tandem mass spectrometry (tandem-MS NBS).

The present invention is particularly useful for detection of proximal markers like glutamine, which serve as diagnostic marker for proximal urea cycle disorders defects. The current invention helps to detect additional markers like glutamine which helps to discriminate between the proximal and distal urea cycle defects, as such markers could be products of the proximal enzymes and a substrate for the distal enzymes. Prompt replacement of glutamine and other amino acids is then possible once it is determined whether the defect is in a proximal or distal urea cycle disorder. Dosing of IV glutamine in proximal urea cycle disorders may then be carried out for fast recovery of the subject.

The present disclosure thus provides reliable and sensitive methods and reagent kits to evaluate the predisposition, presence and severity of a broader number of UCDs including OTC deficiency, argininosuccinate synthetase deficiency (citrullinemia), argininosuccinate lyase deficiency (argininosuccinicaciduria), arginase deficiency and hyperammonemia-hyperornithinemia-homocitrullinemia syndrome (HHH), at an improved detection and/or precision level than the methods typically practiced at the present time, by the determination and quantification of a combination of various indicator metabolites in a biological sample.

### SUMMARY OF THE INVENTION

The scope of the present invention is defined by the appended claims.

The present disclosure relates to methods and reagent kits for the simultaneous detection of lysine and glutamine from a sample and specifically for measuring the levels of glutamine in a sample obtained from a subject. This method is particularly useful for newborn screening (NBS) and particularly for detecting urea cycle disorders or deficiencies (UCDs), OTC deficiency, and/or hyperammoneamia, and/or argininosuccinic aciduria. Such methods and kits are also useful for detecting and/or quantifying further metabolites including amino acids, organic acids, a plurality of carnitines, and/or succinyl acetone in said sample.

The present disclosure also relates to methods and kits for detecting UCDs, hyperammoneamia, and/or argininosuccinic aciduria in newborns, comprising detecting and/or measuring the levels of metabolites, particularly glutamine and/or lysine and glutamine in a sample, such as a dried blood spot obtained from the newborns.

The present disclosure further relates to methods and reagent kits for screening newborns for metabolic disorders, such UCDs, hyperammoneamia, and/or argininosuccinic aciduria, comprising detecting and/or measuring the levels of metabolites, particularly glutamine and/or lysine and glutamine in a sample, such as a dried blood stop obtained from the newborns.

The present disclosure finally provides an internal standard solution comprising at least a lysine stable-isotope labeled internal standard, optionally comprise a glutamine stable-isotope labeled internal standard, and may further comprise additional stable-isotope labeled internal standards.

WO 2015/109263 discloses a method of determining amino acid levels using liquid chromatography - tandem mass spectrometry.

### FIGURES

**Figure 1****:** shows a schematic of the urea cycle.
**Figure 2****:** shows first enzymatic steps of the urea cycle within the mitochondrial matrix.

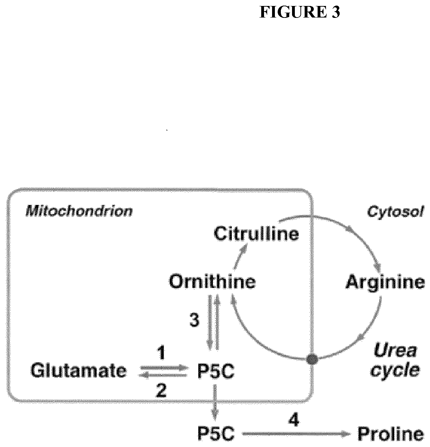

**Figure 3****:** shows the metabolic pathways involved for the synthesis of ornithine, arginine, and proline from glutamate. The enzymes as labeled are (1) pyrroline-5-carboxylase synthase (P5CS), (2) P5C dehydrogenase, (3) ornithine aminotransferase (OAT), (4) P5C reductase.
**Figure 4****:** is a table showing pathological values for the main enzymatic deficiencies within the urea cycle.
**Figure 5****:** is a table providing some control levels of endogenous amino acids.
**Figure 6****:** is a graph showing the linearity of the measurement of glutamine, lysine, arginino succinic acid, and orotic acid and standard solutions of the respective analyte using tandem mass spectrometry.
**Figure 7****:** is a graph showing the linearity of the measurement of glutamine, lysine, arginino succinic acid, and orotic acid and standard solutions of the respective analyte using tandem mass spectrometry.
**Figure 8****:** is a graph showing the linearity of the measurement of glutamine, lysine, arginino succinic acid, and orotic acid and standard solutions of the respective analyte using tandem mass spectrometry.
**Figure 9****:** is a graph showing the linearity of the measurement the sum of lysine and glutamine in dried blood spots (DBS) using ion exchange chromatography.
**Figure 10****:** shows the proposed results of the measurement of the sum of glycine and lysine, as well as measured values from 180 DBS of healthy newborns, measured values from 2 samples of a patient with proven OTC deficiency, and expected range of patients with urea cycle defects (UCDs).
**Figure 11****:** is a table showing the physiological levels of amino acids in plasma (µmol/L) in men, women, adolescents and children.

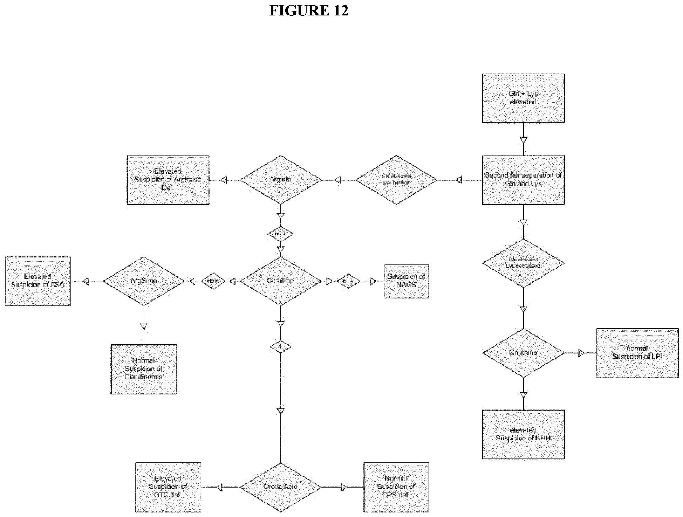

**Figure 12****:** is a flowchart showing the decision tree starting from the detection of elevated sum of glutamine and lysine as early markers to subsequent diagnosis steps for detecting more precisely the type of UCD in the subject.

### DETAILED DESCRIPTION OF THE INVENTION

While the present invention is described in conjunction with various embodiments, it is not intended that the present invention be limited to such embodiments. On the contrary, the present invention encompasses various alternatives, modifications, and equivalents, as will be appreciated by those of skill in the art.

The present disclosure relates to a method and kit for detecting the presence and/or measuring the levels of metabolites in a sample obtained from a subject, by tandem mass spectrometry, said metabolites comprising metabolites having similar mass structure, one of the metabolites being instable, wherein said method comprises the following steps:
(i) extracting said metabolites from the sample with an extraction solution;
(ii) providing, before or after the step (i), a solution comprising a known amount of one or more stable-isotope labeled internal standards corresponding to said metabolites to be detected in the sample,
(iii) ionizing said metabolites and said one or more stable-isotope labeled internal standards to generate ions,
(iv) acquiring the mass to charge (m/z) ratio of said one or more ions in the mass spectrum in the Multiple Reaction Monitoring (MRM) mode, and
(v) determining the sum of said metabolites having similar mass structure in MRM mode,
(vi) detecting and quantifying the amount of said metabolites with stable-isotope labeled internal standard corresponding to one of said metabolites which is stable, and deducing the amount of the other metabolite which is stable.

Indeed, the *in vitro* method from the present invention allows quantifying target metabolites having same isobaric properties, and the quantitative concentration of one or more isobars in a sample can be determined by using the sum or ratios.

In one embodiment of the present disclosure the methods and reagent kits allow quantifying glutamine and lysine. Therefore the present disclosure also relates to a method and reagent kit for detecting the presence and/or measuring the levels of metabolites in a sample obtained from a subject, by tandem mass spectrometry, said metabolites comprising at least glutamine, and optionally lysine, and/or sum of lysine and glutamine, wherein the said method comprises the following steps:
(i) extracting said metabolites from the sample with an extraction solution;
(ii) providing, before or after the step (i), a solution comprising a known amount of one or more stable-isotope labeled internal standards corresponding to said metabolites to be detected in the sample,
(iii) ionizing said metabolites and said one or more stable-isotope labeled internal standards to generate ions,
(iv) acquiring the mass to charge (m/z) ratio of said one or more ions in the mass spectrum in the Multiple Reaction Monitoring (MRM) mode, and
(v) determining the sum of glutamine and lysine in MRM mode,
(vi) detecting and quantifying the amount of glutamine and/or glutamine and lysine with stable-isotope labeled internal standard corresponding to lysine, and optionally glutamine.

As indicated above, said metabolites include isobars, and the quantitative concentration of one or more isobars in a sample can be determined by using the sum or ratios. The methods thus provides for high throughput analysis of metabolites in complex mixtures for unresolved chromatographic peaks and or unstable metabolites. Such methods allow for deconvoluting contributions of a plurality of metabolites in a sample from a mass spectrometer signal, preferably a tandem mass spectrometry signal. Therefore, quantitative concentrations of metabolites, such as, for example, isobars or structural isomers may be obtained. Because of the isobaric natures of glutamine and lysine, sum and or ratios of such isobars can be assessed from the chromatographic signal, subsequently the levels of glutamine may then be deduced by comparing with the stable-isotope labeled internal standard corresponding to lysine or to alternatives to lysine as mentioned above.

Therefore, according to this embodiment, lysine is used to quantify the amount of glutamine. However, a metabolite having a similar structure as that of lysine and being isobaric with glutamine may also be used in the methods and reagent kits of the present disclosure. As indicated above, it is understood that the present methods and kits are not limited to lysine, but that any metabolite having the same mass structure and thus being isobaric to glutamine, may be used as an alternative to lysine having same isobaric properties, and the quantitative concentration of one or more isobars in a sample can be determined by using the sum or ratios, and to quantify the amount of glutamine in the sample of subjects. By way of examples of alternatives for lysine, we can cite glutamate, methionine, methyl-, dimethyl-, trimethy-, hydroxy-, acetyl-, sumoyl-, glocosilated- lysine, as well as precursors of lysine synthesis, such as aspartic acid, etc...

The present disclosure also relates to a method and reagent kit for *in vitro* diagnosis of UCDs, hyperammoneamia, argininosuccinic aciduria, and/or Hyperornithinemia-hyperammonemia-homocitrullinuria (HHH) syndrome in a subject, comprising detecting the presence and/or measuring the levels of metabolites in a sample obtained from a subject, by tandem mass spectrometry, said metabolites comprising at least glutamine and optionally the lysine, and said method comprising the following steps:
(i) extracting the metabolites from the sample with an extraction solution;
(ii) providing, before or after the step (i), a solution comprising a known amount of one or more stable-isotope labeled internal standards corresponding to the metabolites to be detected in the sample,
(iii) ionizing said metabolites and said one or more stable-isotope labeled internal standards to generate ions,
(iv) acquiring the mass to charge (m/z) ratio of said one or more ions in the mass spectrum in the Multiple Reaction Monitoring (MRM) mode,
(v) determining the sum of glutamine and lysine in MRM mode,
(vi) detecting and quantifying the amount of glutamine with stable-isotope labeled internal standard corresponding to lysine, and optionally
(vii) determining whether the level of glutamine as obtained in step (vi) is elevated compared to physiological levels of glutamine.

The present disclosure further relates to *in vitro* methods and reagent kits for screening newborns and/or for detecting in newborns metabolic disorders such as UCDs, hyperammoneamia, argininosuccinic aciduria, and/or Hyperornithinemia-hyperammonemia-homocitrullinuria (HHH) syndrome, comprising detecting the presence and/or measuring the levels of metabolites in a sample obtained from a newborn, by tandem mass spectrometry, said metabolites comprising at least glutamine and optionally lysine, and said method comprising the following steps:
(i) extracting the metabolites from the sample with an extraction solution;
(ii) providing, before or after the step (i), a solution comprising a known amount of one or more stable-isotope labeled internal standards corresponding to the metabolites to be detected in the sample,
(iii) ionizing said metabolites and said one or more stable-isotope labeled internal standards to generate ions,
(iv) acquiring the mass to charge (m/z) ratio of said one or more ions in the mass spectrum in the Multiple Reaction Monitoring (MRM) mode,
(v) determining the sum of glutamine and lysine in MRM mode, and
(vi) detecting and quantifying the amount of glutamine with stable-isotope labeled internal standard corresponding to lysine, and optionally
(vii) determining whether the level of glutamine as obtained in step (vi) is elevated compared to physiological levels of glutamine.

As shown in Figure 1, Urea Cycle Disorders (UCDs) include a variety of genetic defects, which lead to inefficient urea synthesis. UCDs may include one or more enzyme deficiencies within the urea cycle: (a) carbamyl phosphate synthetase deficiency (CPSD or CPS1), (b) N-acetyl glutamate synthetase deficiency (NAGS), (c) ornithine transcarbamylase deficiency (OTCD), (d) argininosuccinic acid synthetase deficiency (ASD), (e) argininosuccinate lyase deficiency or Citrullinemia type 1 (ALD or ASS1), and (f) arginase deficiency (ARG1). Except for OTCD, which is an X-linked genetic disorder, urea cycle disorders are inherited in an autosomal recessive fashion. Each of these diseases represents a defect in the biosynthesis of one of the normally expressed enzymes of the urea cycle and is characterized by signs and symptoms induced by the accumulation of precursors of urea, principally ammonium and glutamine.

Hyperammonemia refers to a clinical condition associated with elevated ammonia levels manifested by a variety of symptoms and signs, including significant central nervous system (CNS) abnormalities. It is crucial to include diagnostic method for early assessment of neonatal predisposition to UCDs, hyperammoneamia, and/or argininosuccinic aciduria in a human neonatal patient, before the physiologic and behavior symptoms become apparent.

Determining the presence and quantifying the amount of glutamine correlates with the presence or absence of urea cycle disorders and/or hyperammoneamia, and/or argininosuccinic aciduria, and/or Hyperornithinemia-hyperammonemia-homocitrullinuria (HHH) syndrome. Methods and reagent kits of the present disclosure are indeed useful for *in vitro* detection of Hyperornithinemia-hyperammonemia-homocitrullinuria (HHH) syndrome characterized by high plasma concentrations of glutamine and ammonia. A characteristic clinical biochemical abnormality in this case is significant elevations in plasma glutamine concentrations. However, as plasma ammonia concentrations return to normal, plasma glutamine concentrations may remain mildly elevated (1.5- to twofold the upper limits of control values). Generally ammonia formed is trapped by Glutamate to form glutamine by the enzyme, Glutamine Synthetase, hence any elevation of glutamine would lead to a higher trapping of ammonia. Hence glutamine would serve as a useful marker for detection and for planning further treatment of HHH by administration of pharmacological agents that can act as glutamine trap in particular and amino acid trap in general, hence diverting nitrogen from urea synthesis to alternatives routes of excretion.

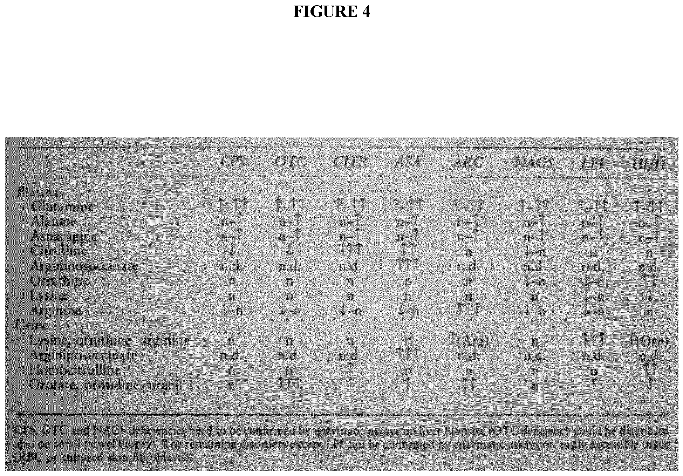

Expected plasmatic physiological levels of glutamine for patients having such deficiencies are well known in the art (Physician's Guide to the Laboratory Diagnosis of Metabolic Diseases, Editors: Nenad Blau, Marinus Duran, Milan E. Blaskovics, K. Michael Gibson, Springer-Verlag Berlin Heidelberg, 2^{nd} Edition 2003, ISBN 978-3-642-62709-5(print) - 978-3-642-55878-8 (Online)). Such levels of glutamine in plasma of healthy subjects are provided in Figures 4, 10 and 11. For example, physiological levels of glutamine in children may range from around 200 to 900µmol/L, or from 250 to 850µmol/L, or from around 330 to 809 µmol/L.

In a preferred embodiment, the extraction step (ii) is performed by using an extraction buffer comprising one or more organic solvents, ionizing step (iii) is performed by delivering said extracted metabolites and standards to an ion source of the mass spectrometer by liquid chromatography system, data acquisition step (iv) is performed with a triple-quadrupole mass spectrometer or high-resolution mass spectrometer, the step (v) is performed by selecting at least one accurate mass-to-charge (m/z) ratio ions corresponding to an at least one calculated mass-to-charge (m/z) ratio of said metabolites present in said sample.

The methods and kits according to the present disclosure are useful for detecting the presence and/or measuring the levels of further metabolites alongside to lysine and glutamine in a sample obtained from a subject, by tandem mass spectrometry, and is thus particularly useful to conduct a plurality of *in vitro* tests to assay further metabolites in the sample of the subject, said method comprising the following steps:
(i) extracting said metabolites from the sample with an extraction solution;
(ii) providing, before or after the step (i), a solution comprising a known amount of one or more stable-isotope labeled internal standards corresponding to the metabolites to be detected in the sample;
(iii) ionizing the extracted metabolites and said one or more stable-isotope labeled internal standards to generate ions;
(iv) acquiring the mass to charge (m/z) ratio of said one or more ions in the mass spectrum in the Multiple Reaction Monitoring (MRM) mode;
(v) determining the sum of glutamine and lysine in MRM mode,
(vi) detecting and quantifying the amount of glutamine with stable-isotope labeled internal standard corresponding to lysine;
(v) determining the presence and/or the levels of said one or more metabolites by analyzing the mass MRM spectrum as obtained in step (iv).

The advantage of *in vitro* methods and kits of the present disclosure is thus that they can be used to determine the presence and/or levels alongside further metabolites which have been commonly used as markers of metabolic diseases, especially in newborns. Said further metabolites that may be further detected in the sample of the subject in addition to the glutamine include without any limitations, amino acids, organic acids, carnitines or a plurality of carnitines, and/or succinylacetone. Elevated amino acids, free carnitine and acylcarnitine levels are examples of metabolites that can be indicative of one or more of several metabolic disorders. Free carnitine and acylcarnitines are markers for disorders that are classified as fatty acid oxidation (FAO) disorders and organic acidurias (OA). Similarly, amino acids are used as markers for several metabolic disorders collectively known as amino acidopathies.

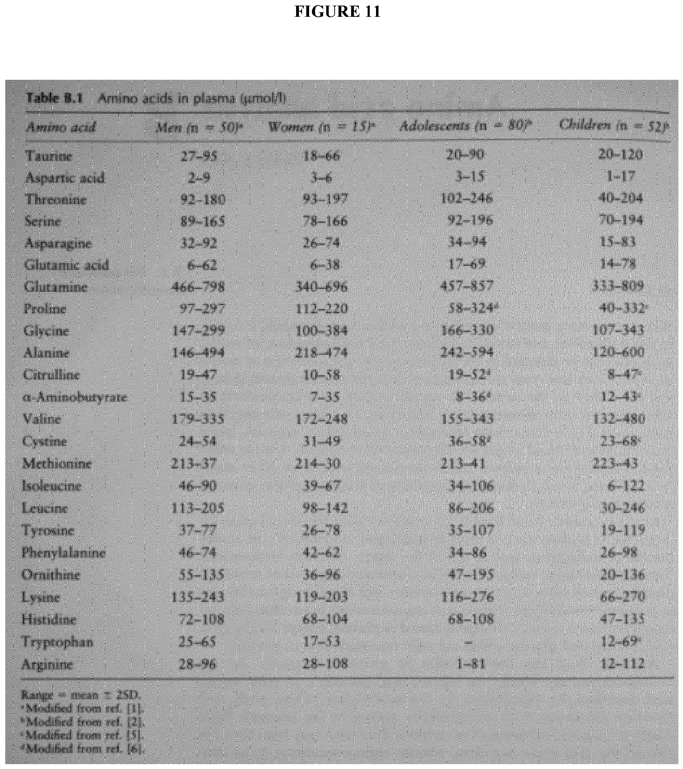

Said further metabolites may comprise arginine, citrulline, argininosuccinate, ornithine, and/or orotic acid. Indeed as showed in the diagnosis decision tree in Figure 11, once an elevated level of glutamine has been detected according to the present invention, a precise determination of the type of UCDs may be further diagnosed.

According to another embodiment, the present disclosure relates to *in vitro* methods and reagent kits for screening newborns and/or for detecting or diagnosing in newborns a suspicion of arginase deficiency (ARG1), comprising detecting the presence and/or measuring the levels of metabolites in a sample obtained from a newborn, by tandem mass spectrometry, said metabolites comprising at least glutamine, arginine and optionally the lysine, and said method comprising the following steps:
(i) extracting the metabolites from the sample with an extraction solution;
(ii) providing, before or after the step (i), a solution comprising a known amount of one or more stable-isotope labeled internal standards corresponding to the metabolites to be detected in the sample,
(iii) ionizing said metabolites and said one or more stable-isotope labeled internal standards to generate ions,
(iv) acquiring the mass to charge (m/z) ratio of said one or more ions in the mass spectrum in the Multiple Reaction Monitoring (MRM) mode,
(v) determining the sum of glutamine and lysine in MRM mode, and
(vi) detecting and quantifying the amount of glutamine with stable-isotope labeled internal standard corresponding to lysine,
(vii) determining whether the amount of glutamine as obtained in step (vi) is elevated compared to physiological levels of glutamine, and
(viii) if the level of glutamine as obtained in step (vii) is elevated, further determining the level of arginine and whether the level of arginine is elevated compared to physiological levels of arginine, thereby allowing to diagnose a suspicion of arginase deficiency.

According to this embodiment, the *in vitro* methods and reagent kits of the present disclosure may also be used for screening newborns and/or for detecting or diagnosing in newborns a suspicion of N-acetyl glutamate synthetase (NAGS), comprising detecting the presence and/or measuring the levels of metabolites in a sample obtained from a newborn, by tandem mass spectrometry, said metabolites comprising at least glutamine, arginine, citrulline, and optionally the lysine, and said method comprising the following steps:
(i) extracting the metabolites from the sample with an extraction solution;
(ii) providing, before or after the step (i), a solution comprising a known amount of one or more stable-isotope labeled internal standards corresponding to the metabolites to be detected in the sample,
(iii) ionizing said metabolites and said one or more stable-isotope labeled internal standards to generate ions,
(iv) acquiring the mass to charge (m/z) ratio of said one or more ions in the mass spectrum in the Multiple Reaction Monitoring (MRM) mode,
(v) determining the sum of glutamine and lysine in MRM mode, and
(vi) detecting and quantifying the elevated level of glutamine with stable-isotope labeled internal standard corresponding to lysine,
(vii) detecting the level of arginine and citrulline with a with stable-isotope labeled internal standard corresponding to arginine and citrulline, respectively, and
(viii) if the level of glutamine as obtained in step (vi) is elevated, and the level of arginine is decreased compared to physiological levels of arginine, further determining whether the level of citrulline is decreased compared to physiological levels of citrulline, thereby suspecting a deficiency of the N-acetyl glutamate synthetase (NAGS).

According to this embodiment, the *in vitro* methods and reagent kits of the present disclosure may further be used for screening newborns and/or for detecting in newborns argininosuccinate aciduria (ASA) and/or citrullinemia, comprising detecting the presence and/or measuring the levels of metabolites in a sample obtained from a newborn, by tandem mass spectrometry, said metabolites comprising at least glutamine, arginine, citrulline, argininosuccinate and optionally the lysine, and said method comprising the following steps:
(i) extracting the metabolites from the sample with an extraction solution;
(ii) providing, before or after the step (i), a solution comprising a known amount of one or more stable-isotope labeled internal standards corresponding to the metabolites to be detected in the sample,
(iii) ionizing said metabolites and said one or more stable-isotope labeled internal standards to generate ions,
(iv) acquiring the mass to charge (m/z) ratio of said one or more ions in the mass spectrum in the Multiple Reaction Monitoring (MRM) mode,
(v) determining the sum of glutamine and lysine in MRM mode, and
(vi) detecting and quantifying the elevated level of glutamine with stable-isotope labeled internal standard corresponding to lysine,
(vii) detecting the level of arginine, citrulline, and argininosuccinate with a with stable-isotope labeled internal standard corresponding to arginine, citrulline, and argininosuccinate, and
(viii) if the level of glutamine as obtained in step (vi) is elevated and the level of arginine is decreased compared to physiological levels of arginine, and further determining whether the level of citrulline is increased compared to physiological levels of citrulline, thereby diagnosing a suspicion of citrullinemia, and further determining whether the level of argininosuccinate is elevated compared to physiological levels of argininosuccinate, thereby suspecting a deficiency of the argininosuccinate aciduria (ASA).

According to this embodiment, the *in vitro* methods and reagent kits of the present disclosure may further be used for screening newborns and/or for detecting or diagnosis in newborns a deficiency of ornithine transcarbamylase deficiency (OTCD) or of carbamoyl phosphate synthetase (CPS), comprising detecting the presence and/or measuring the levels of metabolites in a sample obtained from a newborn, by tandem mass spectrometry, said metabolites comprising at least glutamine, arginine, citrulline, orotic acid and optionally the lysine, and said method comprising the following steps:
(i) extracting the metabolites from the sample with an extraction solution;
(ii) providing, before or after the step (i), a solution comprising a known amount of one or more stable-isotope labeled internal standards corresponding to the metabolites to be detected in the sample,
(iii) ionizing said metabolites and said one or more stable-isotope labeled internal standards to generate ions,
(iv) acquiring the mass to charge (m/z) ratio of said one or more ions in the mass spectrum in the Multiple Reaction Monitoring (MRM) mode,
(v) determining the sum of glutamine and lysine in MRM mode, and
(vi) detecting and quantifying the elevated level of glutamine with stable-isotope labeled internal standard corresponding to lysine,
(vii) detecting the level of arginine, citrulline, and orotic acid with a with stable-isotope labeled internal standard corresponding to arginine, citrulline, and orotic acid, and
(viii) if the level of glutamine as obtained in step (vi) is elevated and the levels of arginine and citrulline are decreased compared to physiological levels of arginine and citrulline, further determining whether the level of orotic acid is increased compared to physiological levels of orotic acid, thereby diagnosing a suspicion of deficiency of the ornithine transcarbamylase (OTCD), or whether the level of orotic acid is normal, thereby diagnosing a suspicion of carbamoyl phosphate synthetase (CPS) deficiency.

According to another embodiment, the present disclosure relates to *in vitro* methods and reagent kits for screening newborns and/or for detecting or diagnosis in newborns a suspicion of Lysinuric protein intolerance (LPI) or Hyperornithinemia-hyperammonemia-homocitrullinuria (HHH), comprising detecting the presence and/or measuring the levels of metabolites in a sample obtained from a newborn, by tandem mass spectrometry, said metabolites comprising at least glutamine, lysine, and ornithine, and said method comprising the following steps:
(i) extracting the metabolites from the sample with an extraction solution;
(ii) providing, before or after the step (i), a solution comprising a known amount of one or more stable-isotope labeled internal standards corresponding to the metabolites to be detected in the sample,
(iii) ionizing said metabolites and said one or more stable-isotope labeled internal standards to generate ions,
(iv) acquiring the mass to charge (m/z) ratio of said one or more ions in the mass spectrum in the Multiple Reaction Monitoring (MRM) mode,
(v) determining the sum of glutamine and lysine in MRM mode, and
(vi) detecting and quantifying the amount of glutamine and lysine with stable-isotope labeled internal standard corresponding to lysine,
(vii) determining whether the amount of glutamine as obtained in step (vi) is elevated and whether the level of lysine is decreased as compared to physiological levels of glutamine and lysine;
(viii) if the level of glutamine as obtained in step (vii) is elevated and level of lysine is decreased compared to physiological levels, further determining whether the level of ornithine is elevated compared to physiological levels of ornithine, thereby allowing to diagnose a suspicion of Hyperornithinemia-hyperammonemia-homocitrullinuria (HHH), or whether the level of ornithine is normal, thereby allowing to diagnose a suspicion of a Lysinuric protein intolerance (LPI).

Physiological levels of arginine, citrulline, argininosuccinate, orotic acids, and ornithine are well known in the art. Those are inter alia described in Physician's Guide to the Laboratory Diagnosis of Metabolic Diseases, Editors: Nenad Blau, Marinus Duran, Milan E. Blaskovics, K. Michael Gibson, Springer-Verlag Berlin Heidelberg, 2nd Edition 2003, ISBN 978-3-642-62709-5(print) - 978-3-642-55878-8 (Online)). Such levels of arginine in plasma of healthy subjects are provided in Figure 11.

Further metabolites may comprise amino acids such as aspartate, serine, proline, phenyalanine, tyrosine, methionine, valine, leucine, glycine, serine, homocitrulline, N-acetyl glutamate, asparagine, alanine, and/or 5-oxoproline. They may also comprise succinylacetone, or organic acid such as.

Said further metabolites to be detected in addition to glutamine may comprise a plurality of carnitines such as acetyl carnitine, propionylcarnitine, butyrylcarnitine, isovalerylcarninitine, glutarylcarnitine, hexanoylcarnitine, octanoylcarnitine, decanoylcarnitine, lauroylcarnitine, myristoylcarnitine, palmitoylcarnitine, and/or stearoylcarnitine.

In particular, said further metabolites may comprise N-acetly glutamate, proline and homocitrulline, as it has been reported so far that a low level of N-acetyl glutamic acid correlated with a deficiency of the NAGS enzyme, or that low level of proline correlated with a deficiency of the pyrroline-5-carboxylase synthase (P5CS), or again that homocitrulline detection in blood correlate with the hyperornithinemia-hyperammonemia-homocitrullinuria (HHH) syndrome.

Preferably said further metabolites comprise argininosuccinate, succinylacetone, arginine, aspartate, serine, proline, N-acetyl glutamic acid, homocitrulline, 5-oxoproline, and orotic acid.

Methods and kits of the present disclosure thus advantageously provide a more complete metabolic profile allowing for a more reliable and efficient *in vitro* diagnosis or prediction of UCDs, hyperammoneamia, and/or argininosuccinic aciduria in a subject, since the accumulation of glutamine, which is an early marker of the urea cycle is now detected in a biological sample of a subject alongside to further metabolites indicative of UCDs, hyperammoneamia, and/or argininosuccinic aciduria. As shown in the table of Figure 4, glutamine is one of the most important marker as it is indicative of a wide range of UCDs. Therefore, detecting and measuring glutamine as well as other metabolites is crucial for the screening and detection of UCDs.

A metabolic profile as described herein can be useful for monitoring the metabolism of a subject (*e.g*., a mammal such as a human), such as neonate. As a non-limiting example, the methods can be used for determining therapeutic efficacy of a particular treatment. Based on this determination, the subject can be offered additional or alternative therapeutic options. The metabolic profile can also be useful for assessing patient compliance with a particular treatment modality, such as dietary restriction. Therefore, the technology described herein is applicable to screening, diagnosis, prognosis, monitoring therapy and compliance, and any other application in which determining the presence or amount of panels of two or more biomolecules is useful. In a most preferred embodiment, the metabolic disorder in determined in a newborn human.

The present disclosure also relates to *in vitro* method and a kit for determining the amount of one or more free metabolites in a body fluid of an individual by mass spectrometry, the method comprising: (a) separating the one or more metabolites by liquid chromatography (LC); and (b) measuring the amount of the one or more metabolites by mass spectrometry (MS) analysis; wherein the one or more metabolites comprises at least lysine and glutamine, and further metabolites as described above.

In a particularly preferred embodiment, the present disclosure is directed to a direct tandem mass spectrometric analysis of metabolites in dried blood spots without chemical derivatization for neonatal screening. Preferably multiple reaction monitoring technique was applied for tandem mass spectrometric measurements, such that each metabolite had its individual precursor and product ion settings.

Determination of the presence or levels of lysine, glutamate, aspartate, serine, proline, orotic acid or glutamine correlates with the presence or absence of UCD, hyperammoneamia, and/or argininosuccinic aciduria. Preferably, the determined amount or presence of lysine correlates with the presence or absence of argininosuccinic aciduria.

The present disclosure thus provides *in vitro* diagnostic methods, reagents, and kits for early assessment of neonatal predisposition to UCD, hyperammoneamia, and/or argininosuccinic aciduria in a human neonatal patient, which method comprises: a) obtaining a blood sample of said neonatal patient immediately, in particular 1 minute to 6 hours after birth and b) assessing in said blood sample at least one panel of low molecular weight early metabolic markers indicative of predisposition and/or the presence or absence of UCD, hyperammoneamia, and/or argininosuccinic aciduria in said a human neonatal patient, wherein the said metabolic markers include at least glutamine.

Determination of succinylacetone (SUAC) from a sample is also particularly useful. Newborn screening for tyrosinemia type I (Tyr-I) is mandatory to identify infants at risk before life-threatening symptoms occur. The analysis of tyrosine alone is limited, and might lead to false-negative results. Consequently, the analysis of SUAC is needed. According to the present invention, there is no need for a separate derivatization step for SUAC.

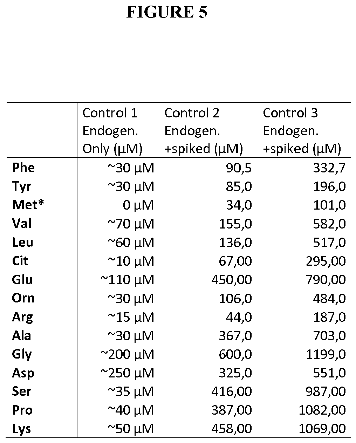

The identity and amount of amino acids in a patient's body fluid is important in a patient's health for a number of reasons. Aberrant amino acid levels can be used to diagnose disease or illness. The presence, absence, identity, amount or modification of an endogenous amino acid as well as its presence and amount in comparison to other amino acids (i.e., the overall profile of free amino acids) are important parameters in assessing a subject's metabolic state. Figure 5 provides some control levels of endogenous amino acids. Aberrant amino acid levels or increased/decreased levels of certain amino acids in comparison to other amino acids can indicate a metabolic disturbance requiring precise and accurate detection and quantitation so that an appropriate intervention can be devised. Therapeutic interventions of metabolic disturbances often include dietary restriction, but can also involve the administration of vitamins and/or other pharmacological agents. Depending on the condition, these treatments may be essential for a patient's survival and optimal mental development. The qualitative and quantitative analysis of free amino acids in biological fluids and tissues is central to the diagnosis and management of a wide variety of metabolic disturbances including primary amino acid enzymopathies (*e.g*., phenylketonuria, maple syrup urine disease) and disorders of amino acid transport (*e.g*., cystinuria). Comprehensive metabolic profiling gives a snapshot of the current physiological state of a subject or experimental organism and, besides uncovering primary metabolic disturbances, is instrumental in evaluating a subject's nutritional status, organ function and compliance with metabolic therapies.

The present disclosure further provides an *in vitro* method for the determination of a disturbance in the metabolic profile of endogenous metabolites in a subject caused by defects in Urea Cycle, the method comprising : (a) measuring the levels of endogenous metabolites in a dried blood sample obtained from the subject, in order to produce a metabolic profile of the endogenous metabolites in that subject; (b) comparing the measured levels of the endogenous metabolites with the corresponding levels of the endogenous metabolites in a biological sample obtained from a control; wherein at least one of the endogenous metabolites is glutamine and at least one of the endogenous metabolites is lysine and optionally the other endogenous metabolites as described above.

More generally, clinically relevant amino acids include all above described proteinogenic amino acids plus non-proteinogenic amino acids plus others including L-alanine, L-arginine, L-asparagine, L-aspartic acid, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, L-valine, gamma-aminobutyric acid (GABA), L-Dopa, hydroxyproline, selenomethionine, phosphoserine, gamma-aminoadipic acid, phosphoethanolamine, sarcosine, β-alanine, taurine, citrulline, beta-aminoisobutyric acid, carnosine, methyl histidine, alpha-aminobutyric acid, anserine, ethanolamine, cystathionine, hydroxylysine, ornithine, argininosuccinate, s-sulfocysteine, homocitrulline, hawkinsin. The methods and kits described herein are useful to identify, detect and/or quantitate any of the listed amino acids alone or in combination.

Also Acylcarnitine profile (ACP) analysis is performed for the biochemical screening of disorders of fatty acid oxidation (FAO) and organic acid metabolism. Inherited FAO disorders are inborn errors of metabolism (IEM) of relatively recent discovery. They may present at any age, from birth to adulthood, frequently leading to life-threatening episodes of metabolic decompensation after a period of inadequate caloric intake and intercurrent illness. Typical manifestations are hypoketotic hypoglycemia, liver disease, skeletal and cardiomyopathy, and sudden unexpected death. Organic acidemias are a more heterogeneous group of IEM. They typically present with recurrent episodes of acute life-threatening illness, hypo- or hypertonia, failure to thrive, and developmental delay. Common acute manifestations include vomiting, lethargy, coma, and seizures. With rare exceptions, the diagnosis of these conditions is almost exclusively a laboratory process, of which acylcarnitine analysis is a key component.

In Organic Acidurias (OA), the metabolic pathways of organic acids are disrupted and thus accumulation of the acids in blood and urine alters the acid-base balance of the body. Resulting modifications or adaptations to intermediary metabolic pathways may cause numerous clinical symptoms, including metabolic acidosis, ketosis, hyperammonemia, failure to thrive, sepsis or coma.

Therefore, as understood by those skilled in the art, metabolic disorders can include, but are not limited to, amino acid disorders, fatty acid oxidation (FAO) disorders, and organic acidurias (OA). Amino acid disorders can include, for example but are not limited to, phenylketonuria (PKU) and other hyperphenylalaninemias, maple syrup urine disease (MSUD), homocystinuria, citrullinemia (types I and II), argininemia, argininosuccinic aciduria (ASA), tyrosinemia (types I and II), homocitrullinuria-hyperornithinemia-hyperammonemia (HHH), methionine adenosyltransferase (MAT) deficiency, biopterin deficiencies, hyperprolinemia, hypermethioninemia, and gyrate atrophy of choroid and retina. Screening for amino acid disorders by MS/MS usually involves making quantitative or semi-quantitative measurements on amino acids.

FAO disorders can include, for example but are not limited to, medium chain acyl-CoA dehydrogenase (MCAD) deficiency, very long chain acyl-CoA dehydrogenase (VLCAD) deficiency, short chain acyl-CoA dehydrogenase (SCAD) deficiency, multiple acyl-CoA dehydrogenase (MAD) deficiency or glutaric academia type II (GA-II), long chain 3-hydroxyacyl-CoA dehydrogenase (LCHAD) deficiency, medium/short chain L-3-hydroxyacyl-CoA dehydrogenase (M/SCHAD) deficiency, trifunctional protein deficiency (TFP), carnitine palmitoyltransferase deficiencies of types I and II (CPT-I, CPT-II), carnitine-acylcarnitine translocase (CACT) deficiency, carnitine transporter deficiency/carnitine uptake defect, short chain 3-ketoacyl-CoA thiolase (SKAT) deficiency, medium chain 3-ketoacyl-CoA thiolase (MCKAT) deficiency, and 2,4-dienoyl-CoA reductase deficiency. Screening for fatty acid disorders by MS/MS usually involves making quantitative or semi-quantitative measurements on acylcarnitines. Free carnitine is not an acylcarnitine, but as those skilled in the art will understand, use of the term "acylcarnitines", in the context of making measurements for the purpose of NBS, often includes free carnitine along with true acylcarnitines.

Furthermore, organic acid disorders can include, for example but are not limited to, 3-methylcrotonyl-CoA carboxylase (3-MCC) deficiency, glutaric acidemia type I (GA-I), methylmalonic acidemias (MMA), propionic acidemia (PA), isovaleric acidemia (IVA), malonic aciduria (MA), multiple carboxylase deficiency (MCD), 2-methyl-3-hydroxybutyrl-CoA Dehydrogenase (MHBD) deficiency, 3-hydroxy-3-methylglutaryl-CoA lyase (HMG) deficiency, 2-methylbutyryl-CoA dehydrogenase (2 MBCD) deficiency, 3-methylglutaconic acidurias (MGA), isobutyryl-CoA dehydrogenase (IBD) deficiency, beta-ketothiolase deficiency (BKT), and ethylmalonic encephalopathy (EE).

The present invention thus provides improved *in vitro* method using tandem mass spectrometry to analyze biological samples, assuring a broader detection of disease conditions of a subject, specifically a newborn subject.

According to the present invention the subject is a subject suspected of having a metabolic disorder, such as a human being, a child, a neonate, a newborn, or a child, preferably a newborn. In particular, such metabolic disorder is an inborn error of metabolism and said subject is a newborn. Preferably, such diagnosis is performed in neonates is between 1 to 5 days of life.

A biological sample containing a bodily fluid, such as dried blood, is subjected to mass spectrometry to yield a plurality of mass spectrometry peaks, at least one of which is analyzed. Optionally, prior to mass spectrometric analysis, the sample is rapidly preprocessed, for example by chromatography, ultrafiltration, electrophoresis or dialysis. Examples of chromatography include ion exchange chromatography, affinity chromatography, hydrophobic chromatography, hydrophilic chromatography and reverse phase chromatography.

The samples that may be used in methods and kits of the present disclosure are biological samples, such as body fluid samples, blood samples and may be in the form of a dried blood spot (DBS).

The dried blood samples may be obtained from a subject or patient by any means. For example, samples may be obtained by pricking the patient's skin (*e.g*., a heel prick) and depositing whole blood on filter paper, or filter card (or Guthrie cards) as one or more spots. The spot or spots may then be punched (*e.g*., with a diameter in the range of about 3/16 inches to 2/16 inches) and placed into a container. For example, different spots may be placed within different wells of a microtiter plate.

Before or after the addition of the isotopic stable internal standards, the dried blood samples are then treated with an extraction solution. Any solution that is able to extract an amount of from dried blood samples may be used for this purpose. Preferred solutions are those that extract the greatest amount of metabolites. Without limitation this includes a variety of organic solvents (with or without water) such as acetonitrile, ethanol, methanol, etc. In one embodiment the extraction solution includes alcohol alone or in combination with water.

A volume of the extraction solution may then be added to each container that includes a dried blood sample. This may be done manually or preferably using automated sample handling equipment. Once the extraction solution had been added to each sample well, the samples are eluted, for example on a shaker table using gentle shaking action. The supernatant is then removed from each container and the remnants of the blood samples are discarded. The solvent in the supernatant is finally removed, for example, by evaporation using heated nitrogen flow.

Such method may or may not require derivatization of the metabolites. The derivatization step is performed by covalently modifying, i.e., by methylation or ester formation, one or more metabolites of the sample, in order to detect and measure said metabolites. The method and kits according to the present invention preferably do not require any derivatization.

The present invention thus also relates to use of novel markers and novel compositions of isotopically enriched standards for detection of UCDs, hyperammoneamia, HHH, and/or argininosuccinic aciduria. According to the present invention, said compositions or solutions of isotopically enriched stable standards comprise at least a standard for lysine, and may comprise additional isotopically stable standards corresponding to one or more metabolites to be further detected in the biological sample of a subject.

According to the invention, a set of individual standards comprising a known amount of one or more stable-isotope labeled internal standards corresponding to the metabolites to be detected is then added to the sample before or after the extraction step, so that the test sample that is eventually analyzed by mass spectrometry comprises isotopically enriched standards.

Said stable-isotope labeled internal standards comprise at least a lysine stable-isotope labeled internal standard, and further comprise one or more stable-isotope labeled internal standards corresponding to alanine, arginine, citrulline, homocitrulline, glycine, leucine, methionine, ornithine, phenylalanine, valine, glutamine, aspartic acid, glutamic acid, N-acetyl glutamate, tyrosine, serine, oxoproline, proline, argininosuccinate, succinylacetone, orotic acid, free carnitine, acetyl carnitine, propionylcarnitine, butyrylcarnitine, isovalerylcarninitine, glutarylcarnitine, hexanoylcarnitine, octanoylcarnitine, decanoylcarnitine, lauroylcarnitine, myristoylcarnitine, palmitoylcarnitine, and/or stearoylcarnitine. More preferably, the set of isotopically labeled standards comprise a lysine stable-isotope labeled internal standard, and further comprises one or more stable-isotope labeled internal standards corresponding to arginine, citrulline, argininosuccinate, orotic acid, and/or ornithine.

The levels of the metabolites in the test sample can be determined by comparing a peak in the one or more mass spectra that correspond to the each metabolite with a peak in the one or more mass spectra that corresponds to the isotopically enriched standard corresponding to said metabolite.

As used herein, isotopic labelling, isotopically-labelled, and other similar terms are used as is understood in the art. Specifically, an isotopically labelled compound is a compound in which at least one atom of known position is enriched with an isotope other than the most abundant naturally-occurring isotope for that element. For example, methane may be ¹³C-isotopically-labelled, and have the structure ¹³CH₄, or deuterium-labelled. Deuterium-labelled methane may refer to a compound in which one or more of the four hydrogen atom positions associated with methane are enriched with ²H (D). Common deuterium-labelled methane structures include CDH₃ and CD₄. Isotopic-labelling refers to isotopic enrichment above natural abundance. Preferably, the isotopic purity at the enriched position is greater than 50%. In embodiments of the present invention, the isotopic purity at the enriched position (s) is preferably greater than 80%. More preferably, the isotopic purity at the enriched position (s) is greater than 90%. In some preferred embodiments, the isotopic purity at the enriched position (s) is greater than 95%. In some preferred embodiments, the isotopic purity at the enriched position (s) is greater than 97%. In some preferred embodiments, the isotopic purity at the enriched position (s) is greater than 98%. In some preferred embodiments, the isotopic purity at the enriched position(s) is greater than 99%. According to the current invention for the internal standards, one or more isotopic labels can be used, and when more than one is used, multiple of the same label (e.g. deuterium) or different labels (e.g. deuterium and ¹³C) can be present. Exemplary isotopically-labeled internal standards are those derivatives that can be clearly differentiated from the isotope peaks of the analyte of interest. Any appropriate isotopic labels can be used including, for example, ²H, ¹³C, ¹⁵N, and ¹⁸O or combination thereof. While not being bound by any theory, the physicochemical behavior of such stable isotopically-labeled derivative with respect to sample preparation and signal generation would be expected to be identical to that of the unlabeled analyte, but clearly differentiable on the mass spectrometer.

Amino acid standards which are labeled with one or more of stable isotopes preferably have three or more of mass difference relative to unlabeled amino acid. By labeling to an unlabeled amino acid with stable isotope so as to bring about 3 or more of mass differences, the influence due to natural existence ratio of the isotope in the unlabeled object may be reduced, and highly precise analysis can be performed. For example, distribution of the natural isotopes in alanine (molecular formula; C₃H₇NO₂, molecular mass 88) are molecular weight 88 (95.8%), 89 (3.74%), 90 (0.34%), and 91 (0.02%), and by making the mass difference into 3 or more, the influence of natural isotopes is avoided.

An isotopically enriched standard solution may comprise at least L-lysine-¹³C₆-¹⁵N₂, and may further comprise on or more of the further standards: selected among ²H₃-aspartate, ¹⁵N-2-¹³C-serine, ²H₃-proline, ¹³C₆-Phenylalanine, ¹³C₆-Tyrosine, ²H₃-Methionine, ²H₈-Valine , ²H₃-Leucine, ²H₂-Citrulline, labelled orotic acid, labelled argininosuccinate, labelled succinylacetone, ²H₉-Carnitine, ²H₃-Acetylcarnitine, ²H₃-Propionylcarnitine, ²H₃-Butyrylcarnitine, ²H₉-Isovalerylcarnitine, ²H₉-Glutarylcarnitine, ²H₃-Hexanoylcarnitine, ²H₃-Octanoylcarnitine, ²H₃-Decanoylcarnitine, ²H₃-Lauroylcarnitine, ²H₃-Myristoylcarnitine, ²H₃-Palmitoylcarnitine, and/or ²H₃-Stearoylcarnitine.

According to the methods and kits of the present disclosure ionizing step (iii) is performed by delivering said extracted metabolites and standards to an ion source of the mass spectrometer. Such ion formation process is a starting point for mass spectrum analysis. Several ionization methods are available and the choice of ionization method depends on the sample to be analyzed. For example, for the analysis of polypeptides a relatively gentle ionization procedure such as electrospray ionization (ESI) can be desirable. For ESI, a solution containing the sample is passed through a fine needle at high potential which creates a strong electrical field resulting in a fine spray of highly charged droplets that is directed into the mass spectrometer. Other ionization procedures include, for example, fast-atom bombardment (FAB) which uses a high-energy beam of neutral atoms to strike a solid sample causing desorption and ionization. Matrix-assisted laser desorption ionization (MALDI) is a method in which a laser pulse is used to strike a sample that has been crystallized in an UV-absorbing compound matrix. Other ionization procedures known in the art include, for example, plasma and glow discharge, plasma desorption ionization, resonance ionization, and secondary ionization.

Electrospray ionization (ESI) has several properties that are useful for the invention described herein. For example, ESI can be used for biological molecules such as polypeptides that are difficult to ionize or vaporize. In addition, the efficiency of ESI can be very high which provides the basis for highly sensitive measurements. Furthermore, ESI produces charged molecules from solution, which is convenient for analyzing reporters that are in solution. In contrast, ionization procedures such as MALDI require crystallization of the sample prior to ionization. Since ESI can produce charged molecules directly from solution, it is compatible with samples from liquid chromatography systems. For example, a mass spectrometer can have an inlet for a liquid chromatography system, such as an HPLC, so that fractions flow from the chromatography column into the mass spectrometer. This in-line arrangement of a liquid chromatography system and mass spectrometer is sometimes referred to as LC-MS. A LC-MS system can be used, for example, to separate un-cleaved or partially cleaved tag reporters from cleaved tag reporters before mass spectrometry analysis. In addition, chromatography can be used to remove salts or other buffer components from the tag reporter sample before mass spectrometry analysis. For example, desalting of a sample using a reversed-phase HPLC column, in-line or off-line, can be used to increase the efficiency of the ionization process and thus improve sensitivity of detection by mass spectrometry. Preferably, the ionizing step (iii) is performed by delivering said extracted metabolites and standards to an ion source of the mass spectrometer by liquid chromatography system.

According to the present invention, the acquisition step (iv) of the mass to charge (m/z) ratio is performed in Multiple Reaction Monitoring (MRM) mode and refers to a tandem spectrometry (MS-MS) experiment where one or more specific products of a selected precursor ion, i.e., a parent ion, a molecular ion or a daughter ion, is monitored.

A variety of configurations of mass spectrometers can be used in a method of the invention. Several types of mass spectrometers are available or can be produced with various configurations. In general, a mass spectrometer has the following major components: a sample inlet, an ion source, a mass analyzer, a detector, a vacuum system, and instrument-control system, and a data system. Difference in the sample inlet, ion source, and mass analyzer generally define the type of instrument and its capabilities. For example, an inlet can be a capillary-column liquid chromatography source or can be a direct probe or stage such as used in matrix-assisted laser desorption. Common ion sources are, for example, electrospray, including nanospray and microspray or matrix-assisted laser desorption. Common mass analyzers include a quadrupole mass filter, ion trap mass analyzer and time-of-flight mass analyzer. Preferably, such data acquisition step (iv) may be performed with a triple-quadrupole mass spectrometer or high-resolution mass spectrometer.

The test sample is scanned using a mass spectrometer to produce one or more mass spectra. Any mass spectrometer that can detect a signal from the extracted metabolites and the standards can be used in the inventive methods. A tandem mass spectrometer is used in certain embodiments because it readily identifies different molecular entities that produce a common fragment when subjected to collision-induced dissociation (CID). In tandem mass spectrometry, two mass analyzers are linked in series via a collision cell. The first mass analyzer (first quadrupole) is used to select an ion of interest (e.g., an ion of a particular mass-to-charge ratio (m/z)). The selected ions are then transferred to a collision cell where they are fragmented by collision with an inert gas (e.g., nitrogen or helium or argon). This process is called collisionally activated dissociation (CAD) and is performed in the Collision Cell of the Mass Spectrometer. Once the precursor ions have fragmented, the second mass analyzer (third quadrupole) is used to either scan and detect all of the produced product ions or to select and detect particular fragment ions.

Tandem mass spectrometry was used to ionize lysine and/or glutamine and several amino acids, fragment the ions, and detect specific peaks that are indicative of the presence of these molecules in the sample. The tandem mass spectrometry detection can be accomplished in a number of ways. In one type of tandem mass spectrometry (commonly performed on triple quadrupole tandem mass spectrometers) ions that fragment to produce common product (fragment) ions can be detected as a class by performing a "precursor ion scan", where by selecting the appropriate mass for the common fragmentation in the Collision Cell, all ion that produce the common fragment ions are detected. This type of scan can be used to detect the acylcarnitines in a sample (precursor ion of m/z 85 scan). In a different form of tandem mass spectrometry, ions that fragment to produce a common neutral loss can be detected as a class by performing a so called neutral loss scan where by setting an appropriate mass offset equal to the common neutral loss between first and third quadrupoles all ions that fragment to produce the specified neutral loss are detected. This type of scan is performed to detect amino acids in a sample (neutral loss of m/z 46 if the metabolites in the extracted sample were amino acids). There is generally a neutral loss scan of m/z 46 were several amino acids are detected from the same sample and a precursor scan of m/z 85 where several acylcarnitines are detected from the sample. In yet another type of tandem mass spectrometry known as multiple reaction monitoring (MRM), a precursor ion of interest is selected in the first quadrupole, fragmented in the collision cell and a specific fragment ion resulting from the collisional activation is selected in the third quadrupole and finally detected.

First and third quadrupoles are fixed to respectively select the corresponding precursor and fragment ion pairs of interest for a predetermined amount of time (a few milliseconds). If additional analytes or metabolites need to be detected, additional detection transitions can be introduced in the experiment. The data from all selected mass transitions can be acquired sequentially to obtain the desired information.

A quadrupole mass analyzer, as well as the other mass analyzers described herein, can be programmed to analyze a defined m/z or mass range. This property of mass spectrometers is useful for the invention described herein. Since the mass range of cleaved tag reporters will be known prior to an assay, a mass spectrometer can be programmed to transmit ions of the projected correct mass range while excluding ions of a higher or lower mass range. The ability to select a mass range can decrease the background noise in the assay and thus increase the signal-to-noise ratio. In addition, a defined mass range can be used to exclude analysis of molecules. Therefore, the mass spectrometer can accomplish an inherent separation step as well as detection and identification of metabolites.

Ion trap mass spectrometry utilizes an ion trap mass analyzer. In these mass analyzers, fields are applied so that ions of all m/z are initially trapped and oscillate in the mass analyzer. Ions enter the ion trap from the ion source through a focusing device such as an octapole lens system. Ion trapping takes place in the trapping region before excitation and ejection through an electrode to the detector. Mass analysis is accomplished by sequentially applying voltages that increase the amplitude of the oscillations in a way that ejects ions of increasing m/z out of the trap and into the detector. In contrast to quadrupole mass spectrometry, all ions are retained in the fields of the mass analyzer except those with the selected m/z. One advantage to ion traps is that they have very high sensitivity, as long as one is careful to limit the number of ions being trapped at one time. Control of the number of ions can be accomplished by varying the time over which ions are injected into the trap. The mass resolution of ion traps is similar to that of quadrupole mass filters, although ion traps do have low m/z limitations.

Time-of-flight mass spectrometry utilizes a time-of-flight mass analyzer. For this method of m/z analysis, an ion is first given a fixed amount of kinetic energy by acceleration in an electric field (generated by high voltage). Following acceleration, the ion enters a field-free or "drift" region where it travels at a velocity that is inversely proportional to its m/z. Therefore, ions with low m/z travel more rapidly than ions with high m/z. The time required for ions to travel the length of the field-free region is measured and used to calculate the m/z of the ion.

One consideration in this type of mass analysis is that the set of ions being studied be introduced into the analyzer at the same time. For example, this type of mass analysis is well suited to ionization techniques like MALDI which produces ions in short well-defined pulses. Another consideration is to control velocity spread produced by ions that have variations in their amounts of kinetic energy. The use of longer flight tubes, ion reflectors, or higher accelerating voltages can help minimize the effects of velocity spread. Time-of-flight mass analyzers have a high level of sensitivity and a wider m/z range than quadrupole or ion trap mass analyzers. Also data can be acquired quickly with this type of mass analyzer because no scanning of the mass analyzer is necessary.

Tandem mass spectrometry can utilize combinations of the mass analyzers described above. Tandem mass spectrometers can use a first mass analyzer to separate ions according to their m/z in order to isolate an ion of interest for further analysis. The isolated ion of interest is then broken into fragment ions (called collisionally activated dissociation or collision induced dissociation) and the fragment ions are analyzed by the second mass analyzer. These types of tandem mass spectrometer systems are called tandem in space systems because the two mass analyzers are separated in space, usually by a collision cell. Tandem mass spectrometer systems also include tandem in time systems where one mass analyzer is used, however the mass analyzer is used sequentially to isolate an ion, induce fragmentation, and then perform mass analysis. Mass spectrometers in the tandem in space category have more than one mass analyzer. For example, a tandem quadrupole mass spectrometer system can have a first quadrupole mass filter, followed by a collision cell, followed by a second quadrupole mass filter and then the detector. Another arrangement is to use a quadrupole mass filter for the first mass analyzer and a time-of-flight mass analyzer for the second mass analyzer with a collision cell separating the two mass analyzers. Other tandem systems are known in the art including reflectron-time-of-flight, tandem sector and sector-quadrupole mass spectrometry.

Mass spectrometers in the tandem in time category have one mass analyzer that performs different functions at different times. For example, an ion trap mass spectrometer can be used to trap ions of all m/z. A series of rf scan functions are applied which ejects ions of all m/z from the trap except the m/z of ions of interest. After the m/z of interest has been isolated, an rf pulse is applied to produce collisions with gas molecules in the trap to induce fragmentation of the ions. Then the m/z values of the fragmented ions are measured by the mass analyzer. Ion cyclotron resonance instruments, also known as Fourier transform mass spectrometers, are an example of tandem-in-time systems.

Several types of tandem mass spectrometry experiments can be performed by controlling the ions that are selected in each stage of the experiment. The different types of experiments utilize different modes of operation, sometimes called "scans," of the mass analyzers. In a first example, called a mass spectrum scan, the first mass analyzer and the collision cell transmit all ions for mass analysis into the second mass analyzer. In a second example, called a product ion scan, the ions of interest are mass-selected in the first mass analyzer and then fragmented in the collision cell. The ions formed are then mass analyzed by scanning the second mass analyzer. In a third example, called a precursor ion scan, the first mass analyzer is scanned to sequentially transmit the mass analyzed ions into the collision cell for fragmentation. The second mass analyzer mass-selects the product ion of interest for transmission to the detector. Therefore, the detector signal is the result of all precursor ions that can be fragmented into a common product ion. Other experimental formats include neutral loss scans where a constant mass difference is accounted for in the mass scans. The use of these different tandem mass spectrometry scan procedures can be advantageous when large sets of metabolites are measured in a single experiment as with multiplex experiments.

In view of the above, those skilled in the art recognize that different mass spectrometry methods, for example, quadrupole mass spectrometry, ion trap mass spectrometry, time-of-flight mass spectrometry and tandem mass spectrometry, can use various combinations of ion sources and mass analyzers which allows for flexibility in designing customized detection protocols. In addition, mass spectrometers can be programmed to transmit all ions from the ion source into the mass spectrometer either sequentially or at the same time. Furthermore, a mass spectrometer can be programmed to select ions of a particular mass for transmission into the mass spectrometer while blocking other ions. The ability to precisely control the movement of ions in a mass spectrometer allows for greater options in detection protocols which can be advantageous when a large number of metabolites, for example, from a multiplex experiment, are being analyzed.

Different mass spectrometers have different levels of resolution, that is, the ability to resolve peaks between ions closely related in mass. The resolution is defined as R=m/delta m, where m is the ion mass and delta m is the difference in mass between two peaks in a mass spectrum. For example, a mass spectrometer with a resolution of 1000 can resolve an ion with a m/z of 100.0 from an ion with a m/z of 100.1. Those skilled in the art will therefore select a mass spectrometer having a resolution appropriate for the analyte(s) to be detected.

Mass spectrometers can resolve ions with small mass differences and measure the mass of ions with a high degree of accuracy. Therefore, metabolites of similar masses can be used together in the same experiment since the mass spectrometer can differentiate the mass of even closely related molecules. The high degree of resolution and mass accuracy achieved using mass spectrometry methods allows the use of large sets of metabolites because they can be distinguished from each other.

The level of metabolites in the test sample is then determined by comparing a peak in the one or more mass spectra that corresponds to the metabolites with a peak in the one or more mass spectra that corresponds to the isotopically enriched standard. In one embodiment, relative peak heights are used. In another embodiment, the areas under the peaks are integrated and compared.

Once the analyte/metabolite level in a test sample or dried blood sample has been determined it can be compared with a range of normal levels. If the level is outside this normal range then the dried blood sample or the patient from whom it was obtained may be referred for further analysis. For example, the test could be repeated with one or more additional blood spots to obtain an average level. Additionally or alternatively the metabolites level could be measured using an alternative method known in the art, e.g., an immunochemical method or a mass spectral method using a serum sample.

In a preferred embodiment the system includes the MRM mode of operation of a triple quadrupole (tandem) mass analyzer. In MRM mode of operation, the first mass analyzing quadrupole is set to select a specific "precursor" ion from the ions passing through the first mass analyzing quadrupole, a second non-mass analyzing quadrupole is used to cause controlled dissociation of the precursor ion, and the third quadrupole (the second mass analyzing quadrupole) is set to select only a specific fragment, or "product", ion of the precursor ion. The precursor and product combination is referred to as the MRM ion pair.

According to some embodiments of the present invention, MRM data can be acquired in several different manners, with the differences being in how the laser is allowed to interact with the sample on the target plate, for example, depending on the ablation mode that is used. A "raster" ablation mode involves the laser beam cutting a straight line swath across one or more samples. To accomplish this "rastering" the laser beam is fired at a non-sample location prior to a sample spot of interest, and the target plate can then be moved continuously to present new samples to the laser impingement point. This means that the laser desorption point will encounter an alternating series of sample/no sample sections of the target plate. The resulting MRM data contains a series of ion signal "peaks", indicated by ion counts per second as a function of mass spectrometer analysis time, with non-zero signal where sample was encountered, interspersed with regions of zero-signal baseline in between sample spots. Depending on laser power, speed of target plate movement under the laser beam, number of metabolites being monitored, and sample composition, it can be typical to acquire the data for a single sample (monitoring one, or several, MRM ion pairs) in approximately 0.25 to 5 seconds for this mode of ablation.

In a "discrete" mode of operation, with the laser in a non-firing state the target plate can be positioned under the laser impingement point and the laser can then be turned on for some specific period of time. While the laser is not firing, the mass spectrometer records a signal of zero (since no ions are generated). When the laser commences firing the laser beam desorbs material from this specific location, creating an MRM signal "peak". The MRM signal intensity rises from zero and quickly maximizes, and it then decreases as the sample is depleted from that specific location on the target plate. The MRM signal level returns to the zero baseline level either by turning off the laser, or by permitting the laser to fire until the sample is completely consumed from the particular location and there is no more sample from which to generate ions. After the laser firing is stopped, the sample plate can then be moved so as to present a new sample location for the next ablation. Depending on laser power, sample composition, and the length of time the laser is fired, it can be typical to acquire the data for a single sample in well under one second for this mode of ablation.

Other ablation modes involve the movement of a sample spot according to some pattern, such that the continually firing laser generates a pattern across the sample spot. This "pattern raster" mode generates a steady stream of ions from a few seconds to several minutes, depending on the form and speed of the pattern within the sample spot. One or more sample spots can be included in such a mode, with proper accommodation when moving from one sample spot to the next. The mass spectrometer method can be constructed to perform a number of different measurements, even including the mixing of mass spectrometer scan types. Pattern rasters are useful for measurements which require ablation times longer than those typically used for MRM analyses with this technique (e.g. precursor or neutral loss scans).

The present disclosure also features liquid chromatography-mass spectrometry methods and kits for the specific, sensitive and rapid detection as well as quantitation of free amino acids in biological fluids following High Pressure Liquid Chromatography (HPLC) separation. High pressure liquid chromatography (HPLC) is a form of column-based chromatography that is routinely used in analytical chemistry to separate, identify or detect and quantify molecules. HPLC utilizes a column that holds chromatographic packing material (stationary phase), a pump that moves the mobile phase(s) through the column, and a detector that detects the abundance of the molecules and shows their retention on the chromatographic column in relation to the elapsed time (retention time). Retention times vary depending on the interactions between the stationary phase, the molecules being analyzed, and the solvent(s) used. A sample containing the analyte or metabolites is injected into the mobile phase manually or by an automated autosampler. The polarity of the analyte, the stationary phase of the column(s) used and the mobile phase(s) determine the retention time of the analyte as well as its separation from interferences and extent of quantifiability. Amino acid separation using HPLC may be performed with any commercially available LC apparatus using automated or manual sample injection and adjustable, consistent and reproducible solvent flow rates.

Columns suitable for liquid chromatography contain packing materials that include very small and usually spherical particles, e.g., silica particles, having a diameter of 3-50 microns and a pore size of 100-1000 angstroms. Commonly, HPLC is performed with a stationary phase attached to the outside surface of such small particles; such stationary phase may provide that surface hydrophobic properties or enable ion change or ion pairing. A chromatographic column typically includes two ports, one inlet port for receiving a sample and one outlet port for discharging an effluent that may or may not include the sample.

In some embodiments of the present invention, the one or more amino acids in a sample enter a column from the inlet port, are then eluted with a solvent or solvent mixture, and eventually discharged at the outlet port. In preferred embodiments, one or more amino acids in a sample enter a column from the inlet port where after the flow across said column is reversed and one or more amino acids are then eluted with a solvent or solvent mixture, and eventually discharged back at the inlet port (flow-reverse). Using a chromatographic column flow-reverse, and specifically using the first chromatographic column of two successive chromatographic columns flow-reverse, proved beneficial in delaying the elution of hydrophilic amino acids and in improving their ionization in the mass detector, leading to increased analytical sensitivity.

Different solvents or solvent mixtures may be selected for eluting the amino acids. For example, liquid chromatography may be performed using a gradient mode with differing amounts of solvents in the mixture, an isocratic mode with continuously fixed amounts of solvents in the mixture or a partially isocratic, partially gradient mixed mode. Suitable solvents and solvent mixtures include sodium or lithium buffers (for cation exchange HPLC) or acetonitrile (for reverse phase HPLC).

The internal diameter of an HPLC column is an important parameter that influences the detection sensitivity and separation selectivity. Column dimensions in preferred embodiments of the present invention include a column internal diameter of 2.1-3.0 mm and a column length of 3-10 cm.

Liquid chromatography is based on the principle that an analyte or metabolite is adsorbed to a stationary phase and eventually desorbed and eluted with the mobile phase into a detection unit for proper detection and/or quantitation. The choice of both stationary and mobile phase greatly influences the success of chromatographic separation.

Reversed phase HPLC (RP-HPLC or RPC) has a non-polar stationary phase and an aqueous, moderately polar mobile phase. One common stationary phase is treated silica. With these stationary phases, retention time is longer for molecules which are more non-polar, while polar molecules elute more rapidly. The mobile phase is generally a binary mixture of water and a miscible polar organic solvent like methanol, acetonitrile or tetrahydrofuran (THF). Reversed phase chromatography is based on partition and is typically used for separations by non-polar differences.

In contrast to reversed phase HPLC, normal phase HPLC (NP-HPLC) uses a polar stationary phase and a non-aqueous, non-polar mobile phase, and works effectively for separating metabolites readily soluble in non-polar solvents. The analyte associates with and is retained by the polar stationary phase until final elution. Typical stationary phases for normal phase chromatography are silica or organic moieties with cyano- and/or amino-functional groups. In NP-HPLC, the most nonpolar molecules elute first and the most polar molecules elute last. The mobile phase consists of a very nonpolar solvent like hexane or heptane mixed with a slightly more polar solvent like isopropanol, ethyl acetate or chloroform. Retention increases, as the amount of nonpolar solvent in the mobile phase increases. NP-HPLC is based on adsorption and is typically employed for the analysis of solutes readily soluble in organic solvents, based on their polar differences such as amines, acids, metal complexes, etc...

Use of ion exchange chromatography may also be within the scope of the invention, since amino acids, by definition, contain at least one amino-group and one carboxyl acid group, they are ionizable and consequently carry a--positive or negative--charge, when the pH of the mobile phase differs from the amino acid's pKa. Below the neutral pH of 7.0, amino acids with primarily basic groups (e.g., amino groups) are positively charged, whereas above the neutral pH of 7.0, amino acids with primarily acidic groups (e.g., carboxylic acid groups) are negatively charged. The 20 proteinogenic amino acids that represent the building blocks of proteins differ in their side-chain groups, which influence the amino acids' chemical reactivity, ionic charge, relative hydrophilicity or hydrophobicity and polarity. Ion-exchange chromatography is a process that allows the separation of ions and polar molecules based on the charge properties of the metabolites. Charged amino acids may be either acidic or basic. The stationary phase surface displays ionic functional groups (R--X) that interact with analyte ions of opposite charge. This type of chromatography is further subdivided into cation exchange chromatography and anion exchange chromatography. The target metabolites (anions or cations) are retained on the stationary phase but can be eluted by increasing concentrations of similarly charged species that will displace the analyte ions from the stationary phase. For example, in cation exchange chromatography, the positively charged analyte could be displaced by the addition of positively charged sodium ions.

Ion-pairing chromatography may also be contemplated for use within the scope of this invention. Similar to ion-exchange chromatography, ion pairing chromatography utilizes the ionizability and charge properties of the metabolites for the chromatographic separation. However, instead of exchanging ions, ion-pairing systems are established using perfluorinated carboxylic acids such as tridecafluoroheptanoic acid (TDFHA) or trifluoroacetic acid (TFA) as mobile phase constituents and a stationary phase that can accept or donate electrons or both. Specifically, ion pairing chromatography can be used to separate ionic metabolites on a reversed-phase column in order to suppress the ionic characteristic of charged organic compounds. Ion pair reagents have a charge opposite of the metabolites and a hydrophobic region to interact with the stationary phase. The charge of the absorbed ion pair reagent interacts electrostatically with the charge of the metabolites. As an example, amines can produce a serious tailing chromatographic peak on a reversed phase column, while addition of an ion pair agent such as trifluoroacetic acid curtails tailing. With advances in column phases and a better selection of ion pair reagents, ion pair chromatography not only sharpens chromatographic peaks but also modulates the retention of ionic metabolites on reverse-phase columns. Typical ion pair reagents include tetra-alkylammonium ions and perfluorinated organic acids. The type of ion pair reagent, the concentration of ion pair reagent, the type of organic modifier in the mobile phase, the concentration (gradient) of the organic modifier, and the proper selection of the columns are critical to a successful ion pair chromatography experiment. In preferred embodiments, ion pair chromatography was used.

The present invention relates to a method for determining the amount of one or more free metabolites in a body fluid of an individual by mass spectrometry, the method comprising: (a) separating the one or more metabolites by liquid chromatography (LC); and (b) measuring the amount of the one or more metabolites by mass spectrometry (MS) analysis; wherein the one or more metabolites comprises of lysine, glutamine, arginine, argininosuccinate, aspartate, serine, proline and orotic acid.

In a particularly preferred embodiment, the present invention is directed to a direct tandem mass spectrometric analysis of analytes in dried blood spots without chemical derivatization for neonatal screening. Preferably multiple reaction monitoring technique was applied for tandem mass spectrometric measurements, such that each metabolite had its individual precursor and product ion settings.

Internal standards for metabolites of interest (or other molecules, *e.g*., biomolecules described herein) detected by a method described herein can be any modification or analog of that analyte molecule that is detectable by mass spectrometry. An internal standard is separately detectable from the molecule based on unique physical characteristics, such as a unique mass or mass-to-charge ratio. A commonly used internal standard for mass spectrometry is a stable isotopically labeled form or chemical derivative of an analyte of interest (e.g., if the analyte was MPP, the internal standard can be an isotopically labeled MPP). For example, stable isotope labeled analogs can be used to quantitate the corresponding analyte of interest using the technique known as isotope dilution mass spectrometry where the analyte and internal standards are processed in the same sample. Internal standards can be designed such that 1) the labeling causes a shift in mass of at least 1 mass unit and 2) that none of the stable isotope labels are located in labile sites to prevent exchange. Labels can be ²H (D), ¹⁵N, ¹³C or ¹⁸O in any combination. The actual location of the labels on the molecule can vary provided the prerequisite 2 (above) is satisfied. Moreover, the position of the labels and the potential change in the mass of the fragment ions can also be used to confirm separation of the internal standard and analytes or metabolites.

In some embodiments, one or more internal standards, representing the types of amino acids that might be present in a particular biological sample, can be added to the biological sample prior to sample preparation and/or sample injection. It is important to note that all internal standards used in the methods and kits of the present disclosure are stable-isotope internal standards. Since stable-isotope standards are not commercially available for every amino acid of interest, the present disclosure is based on a novel combination of stable-isotope standards which has not been reported previously.

The present disclosure also relates to a standard solution which is suitable for amino acid analysis, in particular, relates to a standard solution for use as an external or an internal standard substance, and a method for quantitative method of amino acid using the same.

In a preferred embodiment a composition which can be used as a standard solution according to the present disclosure comprises at least one agent selected from components A to D wherein component A comprises of valine, glycine, alanine, arginine, and glutamate, component B comprises of serine, proline, tyrosine, leucine, isoleucine, lysine, methionine, orinithine and citrulline, component C comprises of Carnitine, Acetylcarnitine, Propionylcarnitine, Butyrylcarnitine, Isovalerylcarnitine, Glutarylcarnitine, Hexanoylcarnitine, Octanoylcarnitine, Decanoylcarnitine Lauroylcarnitine, Myristoylcarnitine, Palmitoylcarnitine, Stearoylcarnitine, component D comprises of Succinylacetone, Orotic acid and argininosuccinate.

In a particularly preferred embodiment, the present disclosure provides an internal standard solution comprising at least a lysine stable-isotope labeled internal standard, and optionally comprising a glutamine stable-isotope labeled internal standard, optionally the internal standard preparation further comprises one or more stable-isotope labeled internal standards corresponding to alanine, arginine, citrulline, glycine, leucine, methionine, ornithine, phenylalanine, valine, lysine, aspartic acid, glutamic acid, tyrosine, serine, oxoproline, proline, argininosuccinate, succinylacetone, orotic acid, free carnitine, acetyl carnitine, propionylcarnitine, butyrylcarnitine, isovalerylcarninitine, glutarylcarnitine, hexanoylcarnitine, octanoylcarnitine, decanoylcarnitine, lauroylcarnitine, myristoylcarnitine, palmitoylcarnitine, and/or stearoylcarnitine.

In another preferred embodiment a composition which can be used as a standard solution according to the present disclosure comprises at least one agent selected from components A to D wherein component A comprises of valine, glycine, alanine, arginine, and glutamate, component B comprises of serine, proline, tyrosine, leucine, isoleucine, lysine, methionine, orinithine and citrulline, component C comprises of Carnitine, Acetylcarnitine, Propionylcarnitine, Butyrylcarnitine, Isovalerylcarnitine, Glutarylcarnitine, Hexanoylcarnitine, Octanoylcarnitine, Decanoylcarnitine Lauroylcarnitine, Myristoylcarnitine, Palmitoylcarnitine, Stearoylcarnitine, component D comprises of Succinylacetone, Orotic acid and argininosuccinate wherein one or more atoms of each compound are labeled with a stable isotope.

In another embodiment, the present disclosure provides an *in vitro* method for analyzing metabolites in dried blood sample, the method comprising (i) providing a composition which can be used as an internal standard solution comprising of at least one agent selected from components A to D wherein component A comprises of valine, glycine, alanine, arginine, and glutamate, component B comprises of serine, proline, tyrosine, leucine, isoleucine, lysine, methionine, orinithine and citrulline, component C comprises of Carnitine, Acetylcarnitine, Propionylcarnitine, Butyrylcarnitine, Isovalerylcarnitine, Glutarylcarnitine, Hexanoylcarnitine, Octanoylcarnitine, Decanoylcarnitine Lauroylcarnitine, Myristoylcarnitine, Palmitoylcarnitine, Stearoylcarnitine, component D comprises of Succinylacetone, Orotic acid and argininosuccinate wherein one or more atoms of each compound are labeled with a stable isotope; (ii) adding the said standard to a dried blood sample; (iii) analyzing the amino acids and the internal standard in the sample by separation analysis; and (iv) determining the concentration of amino acids in the plasma sample based on the obtained results of the internal standard.

In a preferred embodiment a composition which can be used as a standard solution according to the present disclosure comprises glycine, valine, succinylacetone, argininosuccinate and at least one of leucine, phenylalanine, tyrosine, and citrulline.

The methods described herein involve detecting the ratios or sum or weighted sum two or more amino acids, say for example of lysine and glutamine and one or more additional biological metabolites (*e.g*., amino acids, free carnitine, or acylcarnitines, SUAC, organic acids) where the presence or amount of each biomolecule correlates the presence or absence of a metabolic disorder. The methods described herein can be used quantitatively, if desired, to allow comparison of test sample results with known or a predetermined standard amount of a particular analyte(s) (e.g., by using an internal standard as described above). The methods can also be used qualitatively when a test sample is compared with a reference sample, which can be either a normal reference or metabolic disorder reference. In this format, the relative amount of biomolecules can be indicative of a metabolic disorder. A reference sample, for example, can be from a subject having, not suspected of having, or not at risk of developing a disorder such as a metabolic disorder such as urea cycle disorder.

Generally, a cut-off value for a given biomolecule can vary and would be known in the art for commonly tested metabolites and enzymes. Routine, obvious adaptations of methods known in the art can be used to establish cut-off values for uncommonly tested metabolites. A cut-off value is typically a biomolecule amount, or ratio with another biomolecule, above or below which is considered indicative of a metabolic disorder or cause for retest. Thus, in accordance with the invention described herein a reference level of at least one biomolecule in a particular sample type is identified as a cut-off value, above (or in some cases also below) which there is a significant correlation between the presence (or absence) of the at least one biomolecule and presence (or absence) of a metabolic disorder. It is understood that biomolecule panels can be interpreted as a whole, in parts or on an analyte-by-analyte basis.

Those of skill in the art will recognize that some cut-off values are not absolute in that clinical correlations are still significant over a range of values on either side of the cutoff; however, it is possible to select an optimal cut-off value (e.g. varying H-scores, and the like) of biomolecule for a particular sample type. Cut-off values determined for use in the methods described herein generally are compared with published ranges but can be individualized to the methodology used and patient population. It is understood that improvements in optimal cut-off values could be determined depending on the sophistication of statistical methods used and on the number and source of samples used to determine reference level values for the different biomolecules and sample types. Therefore, established cut-off values can be adjusted up or down, on the basis of periodic re-evaluations or changes in methodology or population distribution. In addition, instrument-specific cut-off values can be used, if desired, for example such as when inter-instrument performance comparability is >10%.

The reference level can be determined by a variety of methods, provided that the resulting reference level accurately provides an amount of each biomolecule above which exists a first group of subjects (e.g., humans) having a different probability of metabolic disorder than that of a second group of subjects having metabolite or enzyme activity amount below the reference level. The reference level can be determined by comparison of biomolecule amount in, *e.g*., populations of subjects (*e.g*., patients) having the same metabolic disorder. This can be accomplished, for example, by histogram analysis, in which an entire cohort of patients are graphically presented, wherein a first axis represents the amount of biomolecule and a second axis represents the number of subjects in the cohort whose sample contain one or more biomolecules at a given amount. Two or more separate groups of subjects can be determined by identification of subsets populations of the cohort which have the same or similar levels of biomolecules. Determination of the reference level can then be made based on an amount which best distinguishes these separate groups. The reference level can be a single number, equally applicable to every subject, or the reference level can vary, according to specific subpopulations of subjects. For example, older subjects can have a different reference level than younger subjects for the same metabolic disorder. In addition, a subject with more advanced disease (e.g., a more advanced form of a metabolic disorder) can have a different reference value than one with a milder form of the disease.

According to one embodiment of the present invention, some of the markers are direct measures of a particular chemical substance and some are derived as sums and/or ratios from two or more direct measures, in a preferred embodiment sum of lysine and glutamine are employed as a specific measure for detection of disease conditions like urea cycle disorders and/or hyperammonaemia.

The current method also provides an *in vitro* method for conducting newborn screening by analyzing contributions of a plurality of metabolites utilizing a tandem mass spectrometry signal, said method comprising: obtaining a tandem mass spectrometry signal of a sample comprising a plurality of peak intensities, wherein more than one of said metabolites in said sample contributes to at least one of said peak intensities; providing a model relating said peak intensities to a contribution intensity for each of said metabolites; wherein said model comprises representing said signal as a weighted sum of reference signals which includes measuring the sum of lysine and glutamine, where each reference signal corresponding to one of said metabolites; calculating said contribution intensities of each of said metabolites using said model; providing a calibration curve for at least one of said metabolites relating contribution intensity to concentration; and determining, based on said calibration curve, a concentration of at least one analyte/metabolite and using the results to interpret specific disease states including urea cycle disorders. Mass spectrometry methods according to the present invention can also be extended to include detection of variant proteins and polypeptides implicated in serious diseases. For example, many variant or mutant forms of the polypeptide sub-units of hemoglobin are known to result in various forms of anemia, and many such mutations are of only one amino acid. The basic molecular structure and amino acid sequences of these proteins, and the corresponding mutations in the DNA encoding them, are already of record in the literature.

The preferred method would be targeting specific selected ionised species. This method may be applied with advantage to detect not only variants containing amino acids which differ from the norm, as in the many possible known hemoglobinopathies described hereinafter, but also to detect variants in glycosylation patterns of polypeptides having the normal expected amino acid sequences e.g. as found in the molecule transferrin. Furthermore, the method may be used to detect deletions or additions of one or more amino acid residues from the expected polypeptide sequence. The present invention therefore comprises a method of testing a sample by mass spectrometry, where the ionisation technique produces a multiply-charged spectrum, to detect (a) the presence or absence of a known polypeptide or derivative of a polypeptide, or (b) the presence or absence of a variant of a known polypeptide or derivative of a polypeptide, in which scanning of the sample is targeted to selected ionised species of known mass/charge ratio, the absence of the expected value of mass/charge ratio being indicative of the absence of the known polypeptide or derivative thereof, or the presence of the variant polypeptide or derivative thereof being indicated by a shift in mass/charge ratio from the expected value.

The method of the present invention therefore concentrates data acquisition on a restricted mass/charge range (mass window) to include the normal polypeptide and the variant polypeptide or polypeptides of interest. Targeting in this manner is more reproducible and reveals peaks corresponding to the normal mass/charge ratio and any shifts from the norm due to variants present in samples taken from patients who are either homozygous or heterozygous in this respect. Thus the method may frequently require only one restricted mass window to be targeted. The use of separate windows for the normal and for the variant polypeptide is also possible. Additional mass windows may be used to target other variants or other polypeptides.

According to the current method ionisation technique is used to produce multiply-charged spectrum, to detect (a) the presence or absence of a known polypeptide or derivative of a polypeptide, or (b) the presence or absence of a variant of a known polypeptide or derivative of a polypeptide, in which scanning of the sample is targeted to selected ionised species of known mass/charge ratio, the absence of the expected value of mass/charge ratio being indicative of the absence of the known polypeptide or derivative thereof, or the presence of the variant polypeptide or derivative thereof being indicated by a shift in mass/charge ratio from the expected value.

The method of the present invention can be used to detect any protein variant such as a protein mutation or an abnormal concentration of a wild-type protein. Any inherited disorder leading to variant protein production may therefore be detected using the present invention. Particular protein variants that can be detected with the method of the present invention include haemoglobin variants, albumin, myoblobin, cytochromes, bacterial enzymes and peptide determinants of antigenicity and variant proteins associated with various congenital disorders like for example disorders of glycosylation. It is particularly preferred that the method of the present invention is used to detect clinically important hemoglobin variants such as Hb S, Hb C, Hb D^{Punjab}, Hb O^{Arab}, Hb Lepore, Hb E, delta beta thalassaemia, hereditary persistence of foetal haemoglobin trait (HPFH) and alpha zero thalassemia trait. It is further preferred that the method of the present invention is used to detect the following clinically important haemoglobin variants: S, C, E, D^{Punjab}, and O^{arab.} The protein variant to be detected may be any protein including a glycoprotein. In particular, specific glycoproteins indicative of a metabolic disorder may be detected using the method of the present invention.

Peptide analysis may be conducted simultaneously or sequentially with the metabolite analysis of amino acids, carnitines, acylcarnitines, SUAC, ASA either from the same sample like dried blood spot or from a different sample like different lot of dried blood spot.

The method or kit according to another example can further comprise the step of (v) assaying the enzymatic activity of the polypeptide in the sample, and/or (vi) assaying the amino acid composition of said polypeptide. The step of assaying enzymatic activity may comprise (a) adding a substrate for said enzymatic activity to said sample, and (b) analyzing the sample for the presence or the absence of said substrate and/or for the presence or absence of a product resulting from said enzymatic activity on said substrate. The step of assaying the amino acid composition may comprise (i) adding an endopeptidase like trypsin (ii) analyzing the polypeptides in said sample after the endopeptidase treatment, and (iii) inferring the amino acid composition of said polypeptide.

Also disclosed herein are kits useful for preparing samples for detection and/or measurement (using tandem mass spectrometry) of sum and/or ratio of lysine and glutamine along with multiple other metabolites (*e.g*., other purines and pyrimidines, amino acids, free carnitine, and acylcarnitine) in a dried blood sample. The kits can include one or more internal standards and/or controls for use in subsequent mass spectrometric analysis. For example, the kits can include lysine as an internal standard. The internal standards can each be provided in the kit in a liquid or dried (e.g., lyophilized) form. The kits can include internal standards in a container containing one or more additional controls or internal standards. For example, the kit can include a container with SUAC and/ or ASA control, one or more amino acid controls, and one or more carnitine (*e.g*., free carnitine and acylcarnitines) controls and one or more SUAC and /or argininosuccinate controls, optionally with organic acid controls.

One or more solutions contained in the kit can be stored in, e.g., silanized glass vials. One or more components of the kit can be stored in a container that prevents or minimizes loss of material or evaporation of a solvent. For example, the container can be sealed with a septum.

The kits can include, e.g., dried blood spots useful as a control. For example, the dried blood spot can be enriched with one or more metabolites (*e.g*., one or more metabolites at known concentrations), such as one or more amino acids, free carnitine, or one or more acylcarnitines. The kits can also, optionally, include an extraction solution such as any of the extraction solutions described herein. The extraction solution can contain a C 1-3 linear or branched monoalcohol with at the least 25% of water. The kits can also include one or more solvent solutions containing, *e.g*., acetonitrile or isopropanol. The solvent solutions can also contain water, *e.g*., a solvent solution containing 80% acetonitrile and 20% water.

In a preferred embodiment the present disclosure provides a kit for detecting the presence and/or measuring the levels/amounts of one or more metabolites comprising, lysine and glutamine in a sample obtained from a subject by tandem mass spectrometry, comprising reagents for the detection of said one or more metabolites, comprising internal standard preparation comprising L-lysine-¹³C₆-¹⁵N₂ In a further preferred embodiment, the present disclosure is directed to an internal standard preparation comprising L-lysine-¹³C₆-¹⁵N₂. In yet another preferred embodiment, the present invention is directed to an internal standard preparation comprising ²H₃-glutamate, ²H₃-aspartate, ¹⁵N-2-¹³C-serine, ²H₃-proline, Orotic acid, and/or argininosuccinate.

More specifically the kits according to the disclosure are useful for detecting the presence and quantifying the amounts of further metabolites chosen among argininosuccinate, succinylacetone, aspartate, serine, proline, oxoproline, orotic acid, phenyalanine, tyrosine, methionine, valine, leucine, citrulline, ornithine, arginine, alanine, glycine, free carnitine, acetyl carnitine, propionylcarnitine, butyrylcarnitine, isovalerylcarninitine, glutarylcarnitine, hexanoylcarnitine, octanoylcarnitine, decanoylcarnitine, lauroylcarnitine, myristoylcarnitine, palmitoylcarnitine, and/or stearoylcarnitine.

Further the kits according to the present disclosure comprises internal standard solution comprising one or more stable-isotope labeled internal standards corresponding to alanine, arginine, citrulline, glycine, leucine, methionine, ornithine, phenylalanine, valine, lysine, aspartic acid, glutamic acid, tyrosine, serine, oxoproline, proline, argininosuccinate, succinylacetone, orotic acid, free carnitine, acetyl carnitine, propionylcarnitine, butyrylcarnitine, isovalerylcarninitine, glutarylcarnitine, hexanoylcarnitine, octanoylcarnitine, decanoylcarnitine, lauroylcarnitine, myristoylcarnitine, palmitoylcarnitine, and/or stearoylcarnitine.

In a particularly preferred embodiment, the present disclosure provides reagent kits with an internal standard solution comprising at least a lysine stable-isotope labeled internal standard, and optionally comprising a glutamine stable-isotope labeled internal standard. The reagent kits may alternatively comprise a metabolite having the same mass structure than lysine and thus being isobaric with glutamine. More preferably, the reagent kits according to the present disclosure may comprise one or more of labelled amino acid standards chosen among L-Alanine D₄, L-Arginine HCl (5-¹³C, 4,4,5,5-D₄), L-Citrulline (5,5-D₂), Glycine (2-¹³C, ¹⁵N), L-Leucine (5,5,5-D₃), L-Methionine (S-methyl-D₃), L-Ornithine HCl (3,3,4,4,5,5-D₆), L-Phenylalanine (ring-¹³C₆), L-Valine (D₈), L-Lysine 2HCl (¹³C₆, ¹⁵N₂), L-Aspartic acid (2,3,3-D₃), L-Glutamic acid (2,3,3-D₃), L-Serine (13C3), and/or L-Proline (¹³C₅). Preferred concentrations of amino acids range from 0.2 to 50 µmol/L.

Reagent kits according to the present disclosure also comprise one or more labeled carnitine standards chosen among L-Carnitine (N,N,N-trimethyl-D₉), L-Carnitine, O-Acetyl HCl (N-methyl-D₃), L-Carnitine, O-Propionyl HCl (N-methyl-D₃), L-Carnitine, O-Butyryl HCl (N-methyl-D₃), L-Carnitine, O-Isovaleryl HCl (N,N,N-trimethyl-D₉), L-Carnitine, O-Glutaryl ClO₄ (N-methyl-D₃), L-Carnitine, O-Hexanoyl HCl (N-methyl-D₃), L-Carnitine, O-Octanoyl HCl (N-methyl-D₃), L-Carnitine, O-Decanoyl HCl (N-methyl-D₃), L-Carnitine, O-Dodecanoyl HCl (N-methyl-D₃), L-Carnitine, O-Myristoyl HCl (N,N,N-trimethyl-D₉), L-Carnitine, O-Palmitoyl HCl (N-methyl-D₃), and/or L-Carnitine, O-Octadecanoyl HCl (N-methyl-D₃). Preferred concentrations of (acyl) carnities in the kit reagents of the present invention range from 0.001 to 1 µmol/L.

In a preferred embodiment, the kits according to the disclosure are useful for the *in vitro* diagnosis of metabolic disorders including urea cycle disorder, hyperammoneamia, Hyperornithinemia-hyperammonemia-homocitrullinuria (HHH), and/or argininosuccinic aciduria.

### EXAMPLES

### Example 1: Determination of the levels of glutamine, lysine, arginino succinic acid, and orotic acid

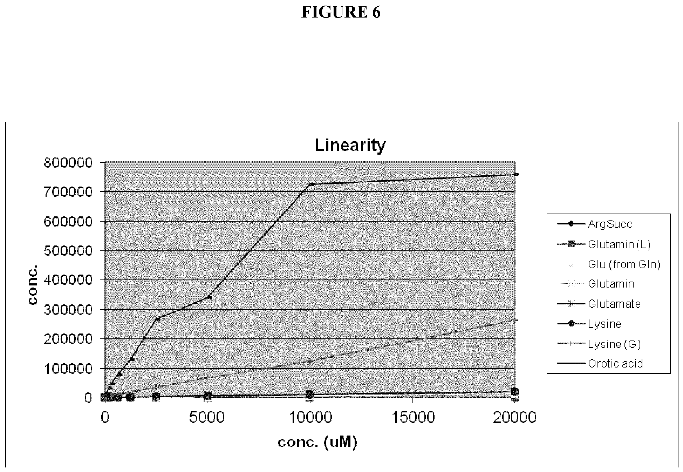

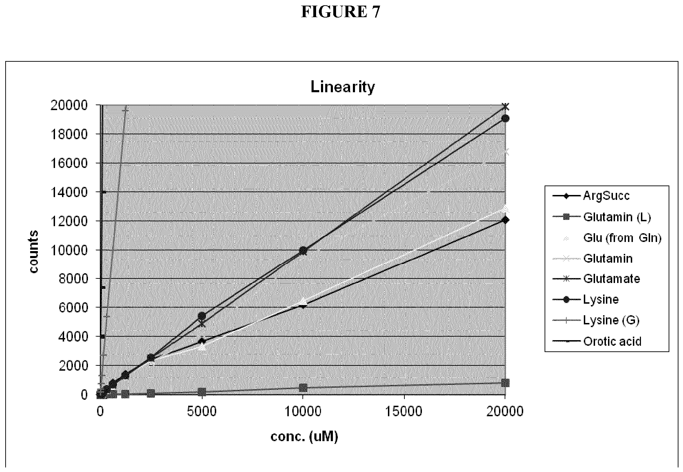

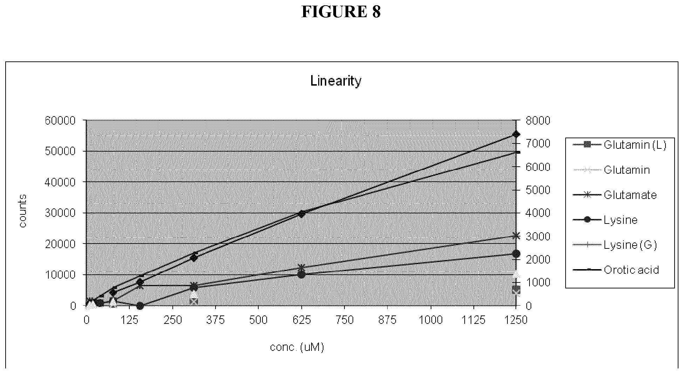

To determine the linearity of the measurement of glutamine, lysine, arginino succinic acid, and orotic acid, standard solutions of the respective analyte up to 20 mM were prepared and 10 µL injected into the tandem mass spectrometer and the respective ions/transitions were measured. (Fig. 6 - 8)

### Example 2: Determination of the levels of glutamine and lysine in dried blood samples

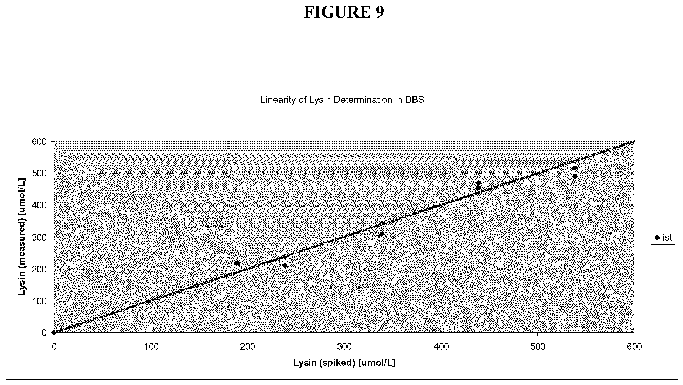

To determine the linearity of the measurement of the sum of lysine and glutamine in dried blood spots (DBS), blood of a healthy donor was spiked with lysine. Endogenous concentrations of lysine and glutamine were determined from an aliquot by standard amino acid determination using ion exchange chromatography. (Fig. 9)

### Example 3: Determination of the sum of glutamine and lysine in dried blood samples

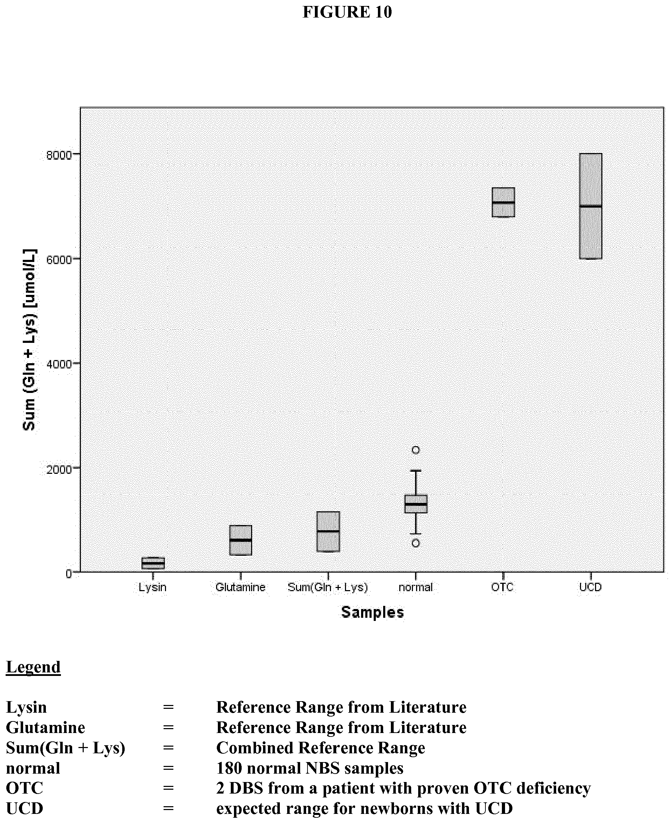

Figure 10 shows the proposed results of the measurement of the sum of glycine and lysine. For lysine and glutamine the reference range of lysine and glycine is plotted, together with the calculated sum of those 2 reference ranges (sum of lower - sum of upper reference range), measured values from 180 DBS of healthy newborns, measured values from 2 samples of a patient with proven OTC deficiency, expected range of patients with urea cycle defects (UCD).

## Claims

1. A method of detecting the presence and/or measuring the levels of glutamine in a sample obtained from a subject, by tandem mass spectrometry, said method comprising the following steps:
(i) extracting said glutamine from the sample with an extraction solution;
(ii) providing, before or after the step (i), a solution comprising a stable-isotope labeled lysine internal standard of known concentration,
(iii) ionizing said glutamine and said stable-isotope labeled lysine internal standard to generate ions,
(iv) acquiring the mass to charge (m/z) ratio of said one or more ions in the mass spectrum in the Multiple Reaction Monitoring (MRM) mode, and
(v) determining the sum of glutamine and lysine in MRM mode,
(vi) detecting and quantifying the amount of glutamine with stable-isotope labeled lysine internal standard,
wherein said ionizing step (iii) is performed by delivering said extracted metabolites and standards to an ion source of the mass spectrometer by liquid chromatography system.

2. The method according to claim 1, wherein one or more metabolites comprising amino acids, organic acids, acylcarnitines, a plurality of carnitines, and/or succinylacetone are further detected and quantified in said sample, said method comprising further extracting said metabolites in step (i), providing known amounts of one or more stable-isotope internal standards corresponding to said additional one or more metabolites to be detected and/or measured, ionizing said additional one or more metabolites, and wherein said method further comprise a step (v) for determining the presence and/or the amounts of said one or more metabolites by analyzing the mass MRM spectrum as obtained in step (iv).

3. The method according to claim 2, wherein said amino acids comprise aspartate, serine, proline, phenyalanine, tyrosine, methionine, valine, leucine, citrulline, homocitrulline, ornithine, arginine, alanine, N-acetyl glutamate, glycine, argininosuccinate and/or oxoproline.

4. The method of claim 2 wherein said organic acid comprises orotic acid, or said acyl carnitines or plurality of carnitines comprise free carnitines, acetyl carnitine, propionylcarnitine, butyrylcarnitine, isovalerylcarninitine, glutarylcarnitine, hexanoylcarnitine, octanoylcarnitine, decanoylcarnitine, lauroylcarnitine, myristoylcarnitine, palmitoylcarnitine, and/or stearoylcarnitine.

5. The method according to any one of the claims 2 to 4, wherein said one or more stable-isotope labeled internal standards comprise at least a lysine stable-isotope labeled internal standard, and further comprise one or more stable-isotope labeled internal standards corresponding to alanine, arginine, citrulline, glycine, leucine, methionine, ornithine, phenylalanine, valine, lysine, aspartic acid, glutamic acid, tyrosine, serine, oxoproline, proline, argininosuccinate, succinylacetone, orotic acid, free carnitine, acetyl carnitine, propionylcarnitine, butyrylcarnitine, isovalerylcarninitine, glutarylcarnitine, hexanoylcarnitine, octanoylcarnitine, decanoylcarnitine, lauroylcarnitine, myristoylcarnitine, palmitoylcarnitine, and/or stearoylcarnitine.

6. The method of claim 5, wherein said one or more stable-isotope labeled internal standards are dried.

7. A method of diagnosing one or more metabolic deficiencies selected among urea cycle disorders (UCDs), and/or hyperammoneamia, Hyperornithinemia-hyperammonemia-homocitrullinuria (HHH), and/or argininosuccinic aciduria, comprising the steps of:
(i) extracting said glutamine from a sample from a subject with an extraction solution;
(ii) providing, before or after the step (i), a solution comprising a stable-isotope labeled internal standard corresponding to lysine of known concentration,
(iii) ionizing said glutamine and said stable-isotope labeled internal standards corresponding to lysine to generate ions,
(iv) acquiring the mass to charge (m/z) ratio of said one or more ions in the mass spectrum in the Multiple Reaction Monitoring (MRM) mode, and
(v) determining the sum of glutamine and lysine in MRM mode,
(vi) detecting and quantifying the amount of glutamine with stable-isotope labeled internal standard corresponding to lysine,
wherein an elevated level of glutamine as obtained in step (vi) is detected compared to physiological level of glutamine.

8. The method according to any one of the preceding claims, wherein said sample is a body fluid sample, preferably a blood sample, and more preferably a dried blood sample, and/or wherein said subject is a subject suspected of having a metabolic disorder, such as a neonate, a newborn, or a child, preferably a newborn.

9. The method according to any one of the preceding claims, wherein said data acquisition step (iv) is performed with a triple-quadrupole mass spectrometer or high-resolution mass spectrometer, and/or wherein the step (v) is performed by selecting at least one accurate mass-to-charge (m/z) ratio ions corresponding to an at least one calculated mass-to-charge (m/z) ratio of said one or more metabolites present in said sample.

10. The method of any one of the preceding claims, wherein the determined amount or presence of glutamine correlates with the presence or absence of urea cycle disorder, and/or hyperammoneamia, and/or Hyperornithinemia-hyperammonemia-homocitrullinuria (HHH), and/or argininosuccinic aciduria.

11. The method according to the preceding claims, further comprising either simultaneously or sequentially, polypeptide analysis, said polypeptide comprising a polypeptide selected among hemoglobin, haemoglobin variants, albumin, myoblobin, cytochromes, bacterial enzymes, peptide determinants of antigenicity, glycoproteins and variant proteins associated with various congenital disorders wherein said method further comprise the step of (v) assaying the enzymatic activity of the polypeptide in the sample, and/or (vi) assaying the amino acid composition of said polypeptide.

12. The method according to claim 11, wherein assaying enzymatic activity in said sample comprises (a) adding a substrate for said enzymatic activity to said sample, and (b) analyzing the sample for the presence or the absence of said substrate and/or for the presence or absence of a product resulting from said enzymatic activity on said substrate, and/or wherein assaying the amino acid composition, comprises (i) adding an endopeptidase (ii) analyzing the polypeptides in said sample after the endopeptidase treatment, and (iii) inferring the amino acid composition of said polypeptide.

13. The method according to claim 11 or 12, where ionisation technique is used to produce multiply-charged spectrum, to detect (a) the presence or absence of a polypeptide or derivative of a polypeptide, or (b) the presence or absence of a variant of a polypeptide or derivative of a polypeptide, in which scanning of the sample is targeted to selected ionised species of known mass/charge ratio, the absence of the expected value of mass/charge ratio being indicative of the absence of the known polypeptide or derivative thereof, or the presence of the variant polypeptide or derivative thereof being indicated by a shift in mass/charge ratio from the expected value.

## Patentansprüche

1. Verfahren zum Detektieren der Präsenz und/oder Messen der Niveaus von Glutamin in einer Probe, gewonnen von einem Subjekt, durch Tandem-Massenspektrometrie, wobei das Verfahren die folgenden Schritte umfasst:
(i) Extrahieren des Glutamins aus der Probe mit einer Extraktionslösung;
(ii) Bereitstellen, vor oder nach dem Schritt (i), einer Lösung, umfassend einen stabil-isotopenmarkierten internen Lysin-Standard von bekannter Konzentration,
(iii) Ionisieren des Glutamins und des stabil-isotopenmarkierten internen Lysin-Standards zum Generieren von Ionen,
(iv) Erfassen des Masse-zu-Ladung (m/z)-Verhältnisses des einen oder mehreren Ionen in dem Massenspektrum in dem Multiple Reaction Monitoring (MRM)-Modus, und
(v) Bestimmen der Summe von Glutamin und Lysin im MRM-Modus,
(vi) Detektieren und Quantifizieren der Menge von Glutamin mit dem stabil-isotopenmarkierten internen Lysin-Standard,
wobei der Ionisierungsschritt (iii) ausgeführt wird durch das Liefern der extrahierten Metaboliten und Standards an eine Ionenquelle des Massenspektrometers durch ein Flüssigkeitschromatographiesystem.

2. Verfahren nach Anspruch 1, wobei ein oder mehrere Metaboliten, umfassend Aminosäuren, organische Säuren, Acylcarnitine, eine Vielzahl von Carnitinen und/oder Succinylaceton weiter in der Probe detektiert und quantifiziert werden, wobei das Verfahren weiter das Extrahieren der Metaboliten in Schritt (i) umfasst, das Bereitstellen bekannter Mengen von einem oder mehreren stabil-isotopenmarkierten internen Lysin-Standards gehörig zu dem einen oder mehreren zusätzlichen zu detektierenden und/oder zu messenden Metaboliten, Ionisieren des zusätzlichen einen oder mehreren Metaboliten und wobei das Verfahren weiter einen Schritt (v) zum Bestimmen der Präsenz und/oder der Mengen des einen oder mehreren Metaboliten durch Analysieren des MRM-Massenspektrums umfasst, wie in Schritt (iv) gewonnen.

3. Verfahren nach Anspruch 2, wobei die Aminosäuren Aspartat, Serin, Prolin, Phenylalanin, Tyrosin, Methionin, Valin, Leucin, Citrullin, Homocitrullin, Ornithin, Arginin, Alanin, N-Acetyl-Glutamat, Glycin, Argininosuccinat und/oder Oxoprolin umfassen.

4. Verfahren nach Anspruch 2, wobei die organische Säure Orotsäure umfasst, oder die Acylcarnitine oder Vielzahl von Carnitinen freie Carnitine, Acetylcarnitin, Propionylcarnitin, Butyrylcarnitin, Isovalerylcarnitin, Glutarylcarnitin, Hexanoylcarnitin, Octanoylcarnitin, Decanoylcarnitin, Lauroylcarnitin, Myristoylcarnitin, Palmitoylcarnitin und/oder Stearoylcarnitin umfassen.

5. Verfahren nach einem der Ansprüche 2 bis 4, wobei der eine oder mehrere stabil-isotopenmarkierte interne Standards wenigstens einen stabil-isotopenmarkierten internen Lysin-Standard umfassen und weiter einen oder mehrere stabil-isotopenmarkierte interne Standards umfassen gehörig zu Alanin, Arginin, Citrullin, Glycin, Leucin, Methionin, Omithin, Phenylalanin, valin, Lysine, Asparaginsäure, Glutaminsäure, Tyrosin, Serin, Oxoprolin, Prolin, Argininosuccinat, Succinylaceton, Orotsäure, freiem Carnitin, Acetylcarnitin, Propionylcarnitin, Butyrylcarnitin, Isovalerylcarnitin, Glutarylcarnitin, Hexanoylcarnitin, Octanoylcarnitin, Decanoylcarnitin, Lauroylcarnitin, Myristoylcarnitin, Palmitoylcarnitin und/oder Stearoylcarnitin.

6. Verfahren nach Anspruch 5, wobei der eine oder mehrere stabil-isotopenmarkierte interne Standards getrocknet werden.

7. Verfahren zum Diagnostizieren einer oder mehrerer metabolischer Störungen, ausgewählt aus Harnstoffzyklusstörungen (UCDs) und/oder Hyperammonämie, Hyperornithinämie-Hyperammonämie-Homocitrullinurie (HHH) und/oder Argininosuccinat-Azidurie, umfassend die folgenden Schritte:
(i) Extrahieren des Glutamins aus einer Probe von einem Subjekt mit einer Extraktionslösung;
(ii) Bereitstellen, vor oder nach dem Schritt (i), einer Lösung, umfassend einen stabil-isotopenmarkierten internen Standard gehörig zu Lysin von bekannter Konzentration,
(iii) Ionisieren des Glutamins und des stabil-isotopenmarkierten internen Standards gehörig zu Lysin zum Generieren von Ionen,
(iv) Erfassen des Masse-zu-Ladung (m/z)-Verhältnisses des einen oder mehreren Ionen in dem Massenspektrum in dem Multiple Reaction Monitoring (MRM)-Modus, und
(v) Bestimmen der Summe von Glutamin und Lysin im MRM-Modus,
(vi) Detektieren und Quantifizieren der Menge von Glutamin mit dem stabil-isotopenmarkierten internen Standard gehörig zu Lysin,
wobei ein erhöhtes Niveau von Glutamin, wie in Schritt (vi) gewonnen, verglichen mit dem physiologischen Niveau von Glutamin detektiert wird.

8. Verfahren nach einem der vorhergehenden Ansprüche, wobei die Probe eine Körperflüssigkeitsprobe ist, bevorzugt eine Blutprobe, und bevorzugter eine getrocknete Blutprobe, und/oder wobei das Subjekt ein Subjekt ist, das unter Verdacht steht, eine metabolische Störung zu haben, beispielsweise ein Neonat, ein Neugeborenes, oder ein Kind, bevorzugt ein Neugeborenes.

9. Verfahren nach einem der vorhergehenden Ansprüche, wobei der Datenerfassungsschritt (iv) mit einem Dreifach-Vierfach-Massenspektrometer oder einem Hochauflösungs-Massenspektrometer durchgeführt wird, und/oder wobei der Schritt (v) durch das Auswählen von Ionen mit wenigstens einem akkuraten Masse-zu-Ladung (m/z)-Verhältnis durchgeführt wird, gehörig zu wenigstens einem kalkulierten Masse-zu-Ladung (m/z)-Verhältnis des einen oder mehreren in der Probe vorhandenen Metaboliten.

10. Verfahren nach einem der vorhergehenden Ansprüche, wobei die bestimmte Menge oder Präsenz von Glutamin mit der Präsenz oder Abwesenheit einer Harnstoffzyklusstörung und/oder Hyperammonämie und/oder Hyperornithinämie-Hyperammonämie-Homocitrullinurie (HHH) und/oder Argininosuccinat-Azidurie korreliert.

11. Verfahren nach einem der vorhergehenden Ansprüche, weiter umfassend entweder simultane oder sequenzielle Polypeptidanalyse, wobei das Polypeptid ein Polypeptid umfasst ausgewählt aus Hämoglobin, Hämoglobinvarianten, Albumin, Myoglobin, Cytochromen, bakteriellen Enzymen, Peptid-Determinanten von Antigenität, Glycoproteine und Variantenproteine assoziiert mit verschiedenen kongenitalen Störungen, wobei das Verfahren weiter den Schritt des (v) Prüfens der enzymatischen Aktivität des Polypeptids in der Probe umfasst, und/oder (vi) des Prüfens der Aminosäurezusammensetzung des Polypeptids.

12. Verfahren nach Anspruch 11, wobei das Prüfen von enzymatischer Aktivität in der Probe (a) das Hinzufügen eines Substrats für die enzymatische Aktivität zu der Probe und (b) Analysieren der Probe auf die Präsenz oder Abwesenheit des Substrats und/oder auf die Präsenz oder Abwesenheit eines Produkts resultierend aus der enzymatischen Aktivität auf dem Substrat umfasst, und/oder wobei das Prüfen der Aminosäurezusammensetzung (i) das Hinzufügen einer Endopeptidase, (ii) das Analysieren der Polypeptide in der Probe nach der Endopeptidasenbehandlung und (iii) das Ableiten der Aminosäurezusammensetzung des Polypeptids umfasst.

13. Verfahren nach Anspruch 11 oder 12, wobei Ionisierungstechnik verwendet wird, um mehrfach geladenes Spektrum zu produzieren, um (a) die Präsenz oder Abwesenheit eines Polypeptids oder Derivats eines Polypeptids oder (b) die Präsenz oder Abwesenheit einer Variante eines Polypeptids oder Derivats eines Polypeptids zu detektieren, in welchem das Scannen der Probe auf ausgewählte ionisierte Arten von bekanntem Masse-zu-Ladung-Verhältnis ausgerichtet ist, wobei die Abwesenheit des erwarteten Werts des Masse-zu-Ladung-Verhältnis indikativ für die Abwesenheit des bekannten Polypeptids oder Derivats davon ist, oder die Präsenz des Varianten-Polypeptids oder Derivats davon von einer Abweichung des Masse-zu-Ladung-Verhältnisses von dem erwarteten Wert indiziert wird.

## Revendications

1. Procédé de détection de la présence et/ou de mesure des niveaux de glutamine dans un échantillon obtenu auprès d'un sujet, par spectrométrie de masse en tandem, ledit procédé comprenant les étapes suivantes :
(i) l'extraction de ladite glutamine de l'échantillon avec une solution d'extraction ;
(ii) la fourniture, avant ou après l'étape (i), d'une solution comprenant un étalon interne de lysine marqué avec un isotope stable de concentration connue,
(iii) l'ionisation de ladite glutamine et dudit étalon interne de lysine marqué avec un isotope stable pour générer des ions,
(iv) l'acquisition du rapport de masse sur charge (m/z) desdits un ou plusieurs ions dans le spectre de masse dans le mode de surveillance de réactions multiples (MRM), et
(v) la détermination de la somme de glutamine et de lysine en mode MRM,
(vi) la détection et la quantification de la quantité de glutamine avec un étalon interne de lysine marqué avec un isotope stable,
dans lequel ladite étape d'ionisation (iii) est effectuée en distribuant lesdits métabolites et étalons extraits à une source d'ions du spectromètre de masse par système de chromatographie liquide.

2. Procédé selon la revendication 1, dans lequel un ou plusieurs métabolites comprenant des acides aminés, des acides organiques, des acylcarnitines, une pluralité de carnitines, et/ou de la succinylacétone sont en outre détectés et quantifiés dans ledit échantillon, ledit procédé comprenant en outre l'extraction desdits métabolites à l'étape (i), la fourniture de quantités connues d'un ou plusieurs étalons internes d'isotope stable correspondant auxdits un ou plusieurs métabolites supplémentaires à détecter et/ou mesurer, l'ionisation desdits un ou plusieurs métabolites supplémentaires, et dans lequel ledit procédé comprend en outre une étape (v) de détermination de la présence et/ou des quantités desdits un ou plusieurs métabolites en analysant le spectre MRM de masse tel qu'obtenu à l'étape (iv).

3. Procédé selon la revendication 2, dans lequel lesdits acides aminés comprennent l'aspartate, la serine, la proline, la phénylalanine, la tyrosine, la méthionine, la valine, la leucine, la citrulline, l'homocitrulline, l'ornithine, l'arginine, l'alanine, le N-acétyl glutamate, la glycine, l'argininosuccinate et/ou l'oxoproline.

4. Procédé selon la revendication 2, dans lequel ledit acide organique comprend de l'acide orotique, ou lesdits acylcarnitines ou la pluralité de carnitines comprennent des carnitines libres, de l'acétylcarnitine, de la propionylcarnitine, de la butyrylcarnitine, de l'isovalerylcarnitine, de la glutarylcarnitine, de l'hexanoylcarnitine, de l'octanoylcarnitine, de la décanoylcarnitine, de la lauroylcarnitine, de la myristoylcarnitine, de la palmitoylcarnitine, et/ou de la stéaroylcarnitine.

5. Procédé selon l'une quelconque des revendications 2 à 4, dans lequel lesdits un ou plusieurs étalons internes marqués avec un isotope stable comprennent au moins un étalon interne de lysine marqué avec un isotope stable, et comprennent en outre un ou plusieurs étalons internes marqués avec un isotope stable correspondant à l'alanine, l'arginine, la citrulline, la glycine, la leucine, la méthionine, l'ornithine, la phénylalanine, la valine, la lysine, l'acide aspartique, l'acide glutamique, la tyrosine, la serine, l'oxoproline, la proline, l'argininosuccinate, la succinylacétone, l'acide orotique, la carnitine libre, l'acétylcarnitine, la propionylcarnitine, la butyrylcarnitine, l'isovalerylcarnitine, la glutarylcarnitine, l'hexanoylcarnitine, l'octanoylcarnitine, la décanoylcarnitine, la lauroylcarnitine, la myristoylcarnitine, la palmitoylcarnitine, et/ou la stéaroylcarnitine.

6. Procédé selon la revendication 5, dans lequel lesdits un ou plusieurs étalons internes marqués avec un isotope stable sont séchés.

7. Procédé de diagnostic d'une ou plusieurs carences métaboliques sélectionnées parmi des troubles du cycle de l'urée (UCD), et/ou l'hyperammoniémie, l'hyperornithinémie-hyperammoniémie-homocitrullinurie (HHH), et/ou l'acidurie argininosuccinique, comprenant les étapes de :
(i) extraction de ladite glutamine d'un échantillon d'un sujet avec une solution d'extraction ;
(ii) fourniture, avant ou après l'étape (i), d'une solution comprenant un étalon interne marqué avec un isotope stable correspondant à la lysine de concentration connue,
(iii) ionisation de ladite glutamine et dudit étalon interne marqué avec un isotope stable correspondant à la lysine pour générer des ions,
(iv) acquisition du rapport de masse sur charge (m/z) desdits un ou plusieurs ions dans le spectre de masse dans le mode de surveillance de réactions multiples (MRM), et
(v) détermination de la somme de glutamine et de lysine en mode MRM,
(vi) détection et la quantification de la quantité de glutamine avec un étalon interne marqué avec un isotope stable correspondant à la lysine,
dans lequel un niveau élevé de glutamine tel qu'obtenu à l'étape (vi) est détecté par comparaison à un niveau physiologique de glutamine.

8. Procédé selon l'une quelconque des revendications précédentes, dans lequel ledit échantillon est un échantillon de fluide corporel, de préférence un échantillon sanguin, et avec plus de préférence un échantillon sanguin séché, et/ou dans lequel ledit sujet est un sujet susceptible de présenter un trouble métabolique, tel qu'un nouveau-né, un nourrisson, ou un enfant, de préférence un nourrisson.

9. Procédé selon l'une quelconque des revendications précédentes, dans lequel ladite étape d'acquisition de données (iv) est effectuée avec un spectromètre de masse de type triple quadrupôle ou un spectromètre de masse à haute résolution, et/ou dans lequel l'étape (v) est effectuée en sélectionnant au moins un rapport de masse sur charge (m/z) précis d'ions correspondant à au moins un rapport de masse sur charge (m/z) calculé desdits un ou plusieurs métabolites présents dans ledit échantillon.

10. Procédé selon l'une quelconque des revendications précédentes, dans lequel la quantité ou présence déterminée de glutamine est en corrélation avec la présence ou l'absence de trouble du cycle de l'urée, et/ou l'hyperammoniémie, et/ou l'hyperornithinémie-hyperammoniémie-homocitrullinurie (HHH), et/ou l'acidurie argininosuccinique.

11. Procédé selon l'une quelconque des revendications précédentes, comprenant en outre, soit simultanément soit séquentiellement, une analyse de polypeptide, ledit polypeptide comprenant un polypeptide sélectionné parmi l'hémoglobine, des variantes d'hémoglobine, l'albumine, la myoblobine, des cytochromes, des enzymes bactériens, des déterminants de peptides d'antigénicité, des glycoprotéines et des protéines variantes associées à divers troubles congénitaux, dans lequel ledit procédé comprend en outre l'étape (v) de titrage de l'activité enzymatique du polypeptide dans l'échantillon, et/ou (vi) de titrage de la composition d'acides aminés dudit polypeptide.

12. Procédé selon la revendication 11, dans lequel le titrage de l'activité enzymatique dans ledit échantillon comprend (a) l'ajout d'un substrat pour ladite activité enzymatique audit échantillon, et (b) l'analyse de l'échantillon pour la présence ou l'absence dudit substrat et/ou la présence ou l'absence d'un produit découlant de ladite activité enzymatique sur ledit substrat, et/ou dans lequel le titrage de la composition d'acides aminés comprend (i) l'ajout d'une endopeptidase, (ii) l'analyse des polypeptides dans ledit échantillon après le traitement d'endopeptidase, et (iii) la déduction de la composition d'acides aminés dudit polypeptide.

13. Procédé selon la revendication 11 ou 12, dans lequel une technique d'ionisation est utilisée pour produire un spectre de charges multiples, pour détecter (a) la présence ou l'absence d'un polypeptide ou d'un dérivé d'un polypeptide, ou (b) la présence ou l'absence d'une variante d'un polypeptide ou d'un dérivé d'un polypeptide, dans lequel le balayage de l'échantillon est ciblé sur des espèces ionisées sélectionnées ayant un rapport de masse sur charge connu, l'absence de la valeur prévue du rapport de masse sur charge étant indicative de l'absence du polypeptide connu ou du dérivé de celui-ci, ou la présence du polypeptide variant ou du dérivé de celui-ci étant indiquée par un changement de rapport de masse sur charge par rapport à la valeur prévue.
